# EUROPEAN PATENT APPLICATION

(11) **EP 4 803 077 A1**
(43) Date of publication of application: **09.09.2026**
(21) Application number: 25161438.4
(22) Date of filing: 03.03.2025
(51) Int. Cl.: A61K 9/70, A61K 38/26, A61K 47/12

(54) **COMPOSITION FOR OROMUCOSAL ADMINISTRATION COMPRISING PERMEATION ENHANCERS**

(71) Applicant: LTS Lohmann Therapie-Systeme AG, 56626 Andernach (DE)
(72) Inventor: LODDER-GADACZEK, Julia, 65618 Selters (DE); HENNES, Michael, 53127 Bonn (DE); PRAAß, Janina, 56204 Hillscheid (DE)
(74) Representative: Maiwald GmbH

(57) **Abstract**

The present invention relates to compositions for the oromucosal administration of an active agent comprising permeation enhancers, and oromucosal delivery systems comprising such compositions.

## Description

### TECHNICAL FIELD OF THE INVENTION

The present invention relates to a composition for the oromucosal administration of an active agent comprising permeation enhancers, and oromucosal delivery systems comprising such compositions.

### BACKGROUND OF THE INVENTION

For medicaments, the route of administration plays a decisive role for important aspects such as the convenience for the patient, the pharmacokinetics, possible adverse effects as well as the cost and ease of manufacture. Of all enteral administration routes, the oral administration of a medicament is the most common, convenient and often cost-effective route.

However, the oral administration route is associated with disadvantages for e.g. elderly and/or cancer patients with difficulties in swallowing or for patients with inconstant or varying bioavailability of oral medications due to gastrointestinal-tract motility disturbances. Passage over a prolonged time in different conditions such as a very low pH in the stomach can affect the solubility as well as stability of the active agent, and not all actives are absorbed well across the intestinal epithelium. Many drugs are also extensively metabolized in the liver, which can result in undesirable hepatotoxicity and reduced amount of active reaching the systemic circulation (first-pass effect). The overall bioavailability *via* the oral administration route is thus in many cases reduced.

Due to such reduced bioavailability, alternative administration routes are commonly explored in particular for expensive drugs. For example, GLP-1 medications are typically provided as injectable solutions in a pre-filled pen, as the poor oral bioavailability of GLP-1 receptor agonists requires subcutaneous administration.

GLP-1 receptor agonists (also known as GLP-1 agonists, GLP-1 analogs, or incretin mimetics) represent a class of medications used in treating type 2 diabetes mellitus (T2DM) and obesity. GLP-1 receptor agonists are synthetic protein preparations that have partial or complete amino acid sequence homology with endogenous GLP-1, but are less easily degraded and have longer half-lives. FDA-approved GLP-1 receptor agonists for glycemic control include dulaglutide, exenatide, liraglutide, lixisenatide, semaglutide and tirzepatide. Furthermore, liraglutide, semaglutide and tirzepatide are FDA-approved as pharmacologic treatment for weight loss.

For semaglutide, an oral dosage form is also available (Rybelsus^{®}). The oral semaglutide absorption is enabled by using a tablet formulation comprising salcaprozate sodium (SNAC) as permeation enhancer. However, the oral bioavailability of semaglutide remains poor and is described to be less than 1%. Accordingly, the tablets are for daily administration, providing dosages of 3 mg (initial dose), 7 mg (maintenance dose) or 14 mg (maximum dose). In contrast, an injectable dosage form of semaglutide (Ozempic^{®}) provide dosages of 0.25 mg (initial dose), 0.5 mg (maintenance dose) and 1 or 2 mg (maximum dose) and only need to be administered once weekly.

In general, patient preference is mainly for less frequent dosing regimens, but also for oral dosage forms over injectable medication, in particular as they do not require the use of needles (needle phobia) and do not incite injection-site reactions such as pruritus and erythema. However, the high drug load needed to compensate the poor bioavailability of GLP-1 receptor agonists in oral dosage forms results in an increased potential of adverse effects. The most frequently exhibited adverse effects from GLP-1 receptor agonists are gastrointestinal and include nausea, vomiting, and diarrhea that could lead to an acute kidney injury due to volume contraction. Severe to life-threatening adverse effects further include pancreatitis, hypoglycemia, thyroid tumors and cancer, visual disturbances (diabetic retinopathy) and allergic reactions, while mild to moderate adverse effects such as dizziness, mild tachycardia, headaches, dyspepsia including constipation and taste disturbances may also occur.

There is thus a need for other dosage forms which are more convenient for the patient and preferably may also reduce the occurrence of adverse effects.

The transmucosal administration route is a less common but very attractive alternative route compared to oral administration, since it is non-invasive and provides for the possibility of self-administration. An active agent administered transmucosally reaches systemic circulation by absorption *via* mucosal tissue directly, which enables a rapid onset of action and bypasses the first-pass metabolism, thus preventing the degradation, metabolization and potentially low absorption due to the gastrointestinal passage of the oral administration route. Transmucosal administration encompasses intranasal, oral as well as rectal administration, of which in terms of ease of administration, the oral transmucosal route, also termed oromucosal route, is preferred.

However, the oromucosal route is also associated with challenges. First of all, the surface area of the oral mucus is rather small so that the amount of medication that can be applied is limited. The mucus also presents a delivery barrier, and not all actives are equally able to cross such a barrier. The epithelial cells of the mucus are covered by mucin, a high molecular weight glycoprotein, which is a first, physical barrier to any active. Large molecules tend to interact with the mucin glycoproteins and are at risk of clearance with the mucus (which is continuously produced and secreted) before the epithelial cells are reached, so that smaller molecules are more likely to successfully diffuse through the mucin layer. Once the epithelium is reached, the active can permeate either transcellularly, i.e., through the cells, passing the cell membrane by partitioning, or paracellularly, i.e., between adjacent cells. Hydrophilic actives do not partition and thus are not able to diffuse through the cell membrane, so that the transcellular route is barred. On the other hand, tight junctions between the cells restrict the efficacy of the paracellular route. Active agents for transmucosal routes are therefore typically of small molecular weight, e.g., less than 500 g/mol, and lipophilic, e.g. with a high logP value (P being the octanol-water coefficient).

To overcome the above aspects which severely restrict the choice of actives that can be delivered transmucosally, penetration enhancers are commonly used to enhance drug absorption. However, irritation at the administration site and toxicity (irreversible damage caused to the structural integrity of the epithelium) are often a concern and many penetration enhancers are still under investigation. In addition, formulating appropriate dosage forms for oromucosal delivery is challenging and the penetration enhancers need to be adequate for formulation. The limited surface area of the oral mucosa also restricts the amount of drug and excipients that can be used, so that efficacy in terms of penetration enhancer amount needed as well as the percentage of drug delivered (drug utilization) needs to be considered. Further, as outlined above, drugs that are currently available for transmucosal administration are largely limited in molecular weight and it would be desirable to be able to enhance permeation of large biomolecules. E.g., in the case of GLP-1 agonists, peptides having a molecular weight of 3,000 g/mol or more (e.g., Liraglutide has 3,751 g/mol, Semaglutide has 4,114 g/mol, and Tirzepatide has 4,813 g/mol) are supposed to permeate transmucosally. Up to date, no commercial GLP-1 receptor agonist transmucosal therapeutic system is available.

Thus, there is still room for improvement for penetration enhancers which are able to promote transmucosal penetration of difficult drug candidates such as large biomolecules.

### OBJECTS AND SUMMARY OF THE INVENTION

It is an object of the present invention to provide a composition comprising penetration enhancers improving the transmucosal absorption of active agents.

It is in particular an object of the present invention to provide a composition comprising penetration enhancers improving the transmucosal absorption of active agents which otherwise would be absorbed transmucosally only in low quantities, so as to make these available for transmucosal dosage forms. Typical drug candidates that are of interest in this sense are large biomolecules with a molecular weight of more than 500 g/mol, more than 1,000 g/mol or even more than 3,000 g/mol.

It is likewise an object of the present invention to provide a composition comprising penetration enhancers improving the transmucosal absorption of active agents, so that less active is needed for a therapeutically effective dose. Lowering the amount of active means that the overall size or volume of the medicament can be reduced, which is in particular desirable for oromucosal formulations, since the oral cavity provides only a limited area of application site.

It is also an object of the present invention to provide a composition comprising penetration enhancers improving the transmucosal absorption of active agents, which is at the same time safe and does not provoke any serious irritation at the administration site or irreversible damage to the epithelial structure of the mucosal tissue.

It is another object of the present invention to provide a composition comprising penetration enhancers improving the transmucosal absorption of active agents so that a desired drug release profile including, e.g., a fast onset of action, can be achieved.

It is similarly an object of the present invention to provide a composition comprising penetration enhancers improving the transmucosal absorption of active agents so that the bioavailability is sufficiently high, thus, e.g., preventing adverse effects.

It is a further object of the present invention to provide a composition comprising penetration enhancers improving the transmucosal absorption of active agents, which is appropriate for formulation in an oromucosal dosage form.

These objects and others are accomplished by the present invention, which according to one aspect relates to a composition for the oromucosal administration of an active agent comprising
A) the active agent, and
B) a combination of permeation enhancers comprising
   i) a first permeation enhancer selected from the group consisting of C6 to C12 fatty acids and salts thereof; and
   ii) a second permeation enhancer selected from the group consisting of bile acids and salts thereof.

It has been surprisingly found that by using such a combination of permeation enhancers, i.e., a C6 to C12 fatty acid or a salt thereof as first permeation enhancer and a bile acid or a salt thereof as second permeation enhancer, the permeation properties and active ingredient utilization can be improved for different active agents, including large molecules such as GLP-1 receptor agonists, while skin irritation or toxicity potential is low.

According to certain embodiments of the invention, the composition for the oromucosal administration of an active agent according to the invention is for use in a method of treatment, and in particular for use in a method of treating a human patient.

According to certain embodiments of the invention, the present invention relates to the use of a composition for the oromucosal administration of an active agent according to the invention for the manufacture of a medicament for treatment, and in particular for treating a human patient.

According to certain embodiments of the invention, the invention relates to a method of treatment including oromucosally administering a composition for the oromucosal administration of an active agent according to the invention, and in particular oromucosally administering a composition for the oromucosal administration of an active agent according to the invention to a human patient.

According to other embodiments, the present invention relates to an oromucosal delivery system comprising the composition for the oromucosal administration of an active agent according to the invention.

### DEFINITIONS

Within the meaning of this invention, the term "composition for the oromucosal administration of an active agent" refers to a composition comprising an active agent, which is suitable for application to and for delivering the active agent via the oral mucosa. The composition preferably comprises therapeutically effective amounts of the active agent so that there is no need for combination treatment with a further medicament. The composition can be in any form that is suitable for, e.g, pharmaceutical application, e.g., in solid form including films, powders, granules, tablets etc., in semi-solid form such as creams, ointments, pastes etc., as well as in liquid form such as solutions, dispersions and emulsions.

Oromucosal therapeutic systems, or oromucosal delivery systems (also termed buccal patches by some), consist of one or more thin layers which are applied and adhere to the mucosa of the oral cavity to deliver the drug over a period of time. Dosage forms in the form of thin films for application in the oral cavity are also sometimes referred to as "Oral Thin Film" or OTF, however, OTFs are not necessarily intended to adhere to the mucosa. In an oromucosal therapeutic system, the active is contained in a dissolvable layer, and due to the film adhering to the mucosa, active delivery is achieved by a combination of direct active release from the oromucosal therapeutic system to the mucosa, and by an indirect active delivery *via* dissolution in the saliva.

Within the meaning of this invention, the term "oromucosal therapeutic system" or "oromucosal delivery system" refers to a system by which an active agent is administered to the systemic circulation *via* transmucosal delivery by application to the mucosa of the oral cavity, and refers to the entire individual dosing unit that is applied to the mucosa of a patient, and which comprises a therapeutically effective amount of the active agent in a mucoadhesive layer structure and optionally an additional overlay on top of the active agent-containing mucoadhesive layer structure. The mucoadhesive layer structure may be located on a release liner (a detachable protective layer), thus, the transmucosal therapeutic system may further comprise a release liner. In the sense of the invention, the term "oromucosal therapeutic system" is in particular understood to mean a system providing passive transmucosal delivery excluding active transport as in methods including microporation. Also, in contrast to certain oral thin films which are not necessarily mucoadhesive and which are intended to disintegrate very fast in the saliva (sometimes referred to as "flash wafers"), enteral delivery is entirely unintended in oromucosal therapeutic systems.

Within the meaning of this invention, the term "mucoadhesive layer structure" or "mucoadhesive layer structure containing a therapeutically effective amount of the active agent" refers to the active agent-containing structure providing the area of release for the active agent during administration. Any additional overlay adds to the overall size of the transmucosal therapeutic system but does not add to the area of release. The mucoadhesive layer structure comprises at least one active agent-containing layer.

Within the meaning of this invention, the term "therapeutically effective amount" refers to a quantity of active agent in the transmucosal therapeutic system sufficient to provide, if administered by the transmucosal therapeutic system to a patient, blood levels of a similar range (e.g. of about 10 % to about 1000 % as measured as an AUC) when compared to blood levels obtained in a one-time administration of commercially available approved drug products of the active agent, such as 0.5-8 mg subcutaneous semaglutide or 1.5-14 mg oral semaglutide.

Within the meaning of this invention, the terms "active", "active agent", and the like refer to any substance of interest for administration to a subject to achieve a desired therapeutic or other, e.g., cosmetic effect, in any pharmaceutically and/or regulatorily acceptable chemical and morphological form and physical state. These forms include without limitation active agents in their free, dissociated or any associated form such as hydrates, solvates and so on, as well as in the form of particles. In accordance with the invention, the term "particles" is understood to mean to a solid, particulate material comprising individual particles, the dimensions of which are negligible compared to the material. In particular, the particles are solid, including plastic/deformable solids, including amorphous and crystalline materials.

The active agent, where contained in a medium such as a solvent, may be dissolved or dispersed or in part dissolved and in part dispersed. In the sense of the invention, the term "dispersing" is understood to mean a step or a combination of steps wherein a starting material is not totally dissolved. Dispersing in the sense of the invention comprises the dissolution of a part of the starting material, depending on the solubility of the starting material (e.g. the solubility of the active agent in the coating composition).

Within the meaning of this invention, the terms "GLP-1 receptor agonist", "GLP-1 agonist" and "GLP-1 analog" refer to a group of active agents mimicking the actions of the endogenous incretin hormone GLP-1. GLP-1 is a 30-amino acid polypeptide processed from proglucagon in the endocrine L-cells distributed primarily in the mucosa of the distal part of the small intestine and colon. Together with GIP (glucose-dependent insulinotropic polypeptide), the most import effect of GLP-1 is its ability to potentiate glucose-induced insulin secretion from the pancreas, the so-called incretin effect. Thus, GLP-1 receptor agonists are also referred to as "incretin mimetics". GLP-1 receptor agonists are usually (synthetic) peptides or peptide conjugates, resulting from intricate structural modifications to GLP-1, enabling them to not only replicate the pharmacological functions of GLP-1 but also to render them resistant to degradation which resulting in a prolonged half-life and heightened biological activity. These synthetic protein preparations can exhibit partial or complete amino acid sequence identity with endogenous GLP-1 (in particular of human GLP-1(7-37), the amino acid sequence of which is HAEGTFTSDVSSYLEGQAAKEFIAWLVKGRG). For example, liraglutide shares 97% sequence identity with human GLP-1 (compared to GLP-1(7-37) the lysine residue at position 34 is replaced by arginine and a hexadecanoyl group is attached to the remaining lysine at position 26 via a spacer), and semaglutide shares 94% sequence identity with human GLP-1 (compared to GLP-1(7-37) the alanine residue at position 8 is replaced by diaminoisobutyric acid, the lysine residue at position 34 is replaced by arginine and a stearic diacid chain is attached to the remaining lysine at position 26 via a spacer). In comparison, tirzepatide has less sequence identity to human GLP-1. It is derived from the native sequences of GLP-1, GIP and semaglutide with the incorporation of further distinct residues. As a result, it serves as a dual agonist for both GLP-1 and GIP. Accordingly, the term "GLP-1 receptor agonist" also relate to dual agonists, targeting both the GLP-1 receptor and another specific receptor, such as the GIP receptor, or to triple agonist, simultaneously targeting the GLP-1 receptor and two other specific receptors. Furthermore, the term "GLP-1 receptor agonists" includes small molecules acting as GLP-1 receptor agonists, such as, e.g., orforglipron or danuligpron.

Within the meaning of the invention, the term "sequence identity" refers to the degree of identity of a first amino acid sequence to a second amino acid sequence, and is calculated as a percentage based on a comparison between the two sequences. The sequence identity is determined by a program, which produces a pairwise alignment, and calculates the identity between the two aligned sequences counting both mismatches at a single position and gaps at a single position as non-identical positions. Sequence identity can be calculated from a pairwise alignment of two sequences over the full length of both sequences ("global sequence identity"), or from a pairwise alignment of the local regions of the first sequence and the second sequence that show identity or similarity ("local sequence identity"). If not indicated otherwise, a sequence identity within the meaning of this invention refers to a sequence identity that is calculated from a pairwise alignment taken into account human GLP-1(7-37) and the sequence of the GLP-1 receptor agonist over its full length. An exemplary program for determining a "global sequence identity" is the "Needle" (The European Molecular Biology Open Software Suite, EMBOSS) program (https://www.ebi.ac.uk/Tools/psa/emboss_needle/). Alignments showing the "local sequence identity" can, for example, be produced by the Blast algorithm (NCBI).

Within the meaning of the invention, the term "peptide" refers to a primary sequence of amino acids that are linked by covalent "peptide linkages." In general, a peptide consists of fewer amino acids than a full-length protein, and typically comprises from 2-50 amino acids. A synthetic peptide is a peptide that is produced by artificial means *in vitro* (e.g., was not produced in vivo). A peptide amino acid sequence may also comprise chemical compounds (peptide conjugate). As used herein, the term "amino acid sequence," refers to the primary (i.e., linear) structure of a peptide or protein, wherein the individual amino acids are linked by peptide bonds.

There are two main types of oromucosal therapeutic systems, i.e. those using backing layers, and those without. Active delivery of an open system type oromucosal therapeutic system using no backing layer will always be a combination of direct delivery from the oromucosal therapeutic system through the mucosa at the adhesion site, and indirect delivery *via* dissolution of the active from the oromucosal therapeutic system into the saliva, and from the saliva through the mucosa. The proportion of the different delivery routes depends mainly on factors such as the solubility of the active and the disintegration time of the oromucosal therapeutic system. The higher the solubility and the faster disintegration of the oromucosal therapeutic system, dissolution into the saliva will be favored over direct delivery into the mucosa at the adhesion site. Such an indirect delivery has the huge advantage of providing a practical increase by several factors of the mucosal surface area through which the active is released systemically. Dissolution into the saliva on the other hand means that the active concentration, and thus the final delivered amount is difficult to control, and that there may be a risk of enteral delivery by unintended swallowing of the saliva.

Oromucosal therapeutic systems using a backing layer have a completely different approach, i.e. in such systems, the loss of being restricted in the drug release area (to the actual size of the patch) is accepted in exchange for limiting the delivery route to the direct transmucosal delivery, which can be much better controlled. Thus, in the sense of the invention, a "backing layer" is any layer within a oromucosal therapeutic system which is able to prevent (at least a substantial amount of) the active contained within the oromucosal therapeutic system to be dissolved into the saliva. Such a backing layer can be non-dissolvable, or dissolvable over time. In the latter case, the time the backing layer takes for dissolution is at least as long as (a substantial amount of) the active takes to be delivered through the mucosa.

In this context, it also becomes clear that terms such as "dissolution", "dissolvable", "dissolve" and the like with respect to any of the layers of a oromucosal therapeutic system (e.g. backing layer, active agent-containing layer) and with respect to the film-forming agent when casted into a film, are to be understood very broadly, and not in the strict scientific sense of chemically dissolving a molecule in a solvent. Any transformation of the solid state of the layer concerned to a liquid state, such as dispersing, forming of a suspension, gelling of the film and disintegrating into smaller parts of gel, etc. has to be regarded as "dissolving" in the sense of the present invention, as long as the "dissolved" material is able to freely move around in the liquid (e.g. saliva) so that anything that was present below the layer concerned (i.e. the mucosa if a mucosa-contacting layer was dissolved, or e.g. the active agent-containing layer if a backing layer was dissolved before the active agent-containing layer) becomes accessible to liquid other than the "dissolved" material. In preferred embodiments, the meaning is limited to the usual chemical sense of dissolving a molecule in a solvent. It should be noted that the term "dissolve" with respect to substances *per se,* such as the active agent, or any excipients, will continue to be used in the usual chemical sense of dissolving a molecule in a solvent. E.g., active agent in dissolved form obviously does not include active agent in dispersed form. The film-forming agent *per se* can be present in the coating composition during manufacture of the oromucosal therapeutic system in dissolved form in the common chemical sense (e.g. is not dispersed, in form of small parts of gel, etc.), but where the film-forming agent is casted into a film, "dissolving" such a film also includes gelling of the film and disintegrating into smaller parts of gel.

Within the meaning of the invention, the term "active agent-containing layer" refers to a layer containing the active agent and providing the area of release. As used herein, the active agent-containing layer is the final, solidified layer e.g. obtained after coating and drying the solvent-containing coating composition. The active agent-containing layer may also be manufactured by laminating two or more such solidified layers (e.g. dried layers) of the same composition to provide the desired area weight. The active agent-containing layer may be mucoadhesive (in the form of a mucoadhesive layer) or the oromucosal therapeutic system may comprise an additional mucosa-contacting layer of a mucoadhesive for providing sufficient adhesion. In particular, the active agent-containing layer is a mucoadhesive layer.

Within the meaning of this invention, the term "mucoadhesive" refers to a material that in particular adheres to and upon contact with a mucosa, but which preferably is non-tacky and can be touched e.g. with the fingers and manipulated, e.g. for application into the oral cavity, without unintentionally adhering to the skin of the fingers, when in dry state. A mucoadhesive layer, when in contact with the mucosa, is "self-adhesive", i.e. provides adhesion to the mucosa so that typically no further aid for fixation is needed. The adhesion strength is preferably strong enough that typical movements in the oral cavity are not sufficient to displace a mucoadhesive layer adhered to the mucosa. A "mucoadhesive" layer structure includes a mucoadhesive layer for mucosa contact which may be provided in the form of a mucoadhesive active agent-containing layer or in the form of an additional layer, i.e. a mucoadhesive mucosa-contacting layer. A mucoadhesive overlay may still be employed to advance adhesion.

Within the meaning of this invention, the term "area weight" refers to the dry weight of a specific layer, e.g. of the active agent-containing layer, provided in g/m², as determined after normal drying (expecting a residual moisture content of less than about 10% such as in a range of from about 5% to about 8%) or, preferably, after total drying (expecting a residual moisture content of approx. 0%). The area weight values are subject to a tolerance of ± 10 %, preferably ± 7.5 %, due to manufacturing variability.

If not indicated otherwise "%" refers to wt-% (% by weight).

Within the meaning of the invention, the term "permeation enhancer" refers to a substance which increases the active agent permeability, e.g., by influencing the barrier properties of the epithelial layer such as, e.g., stratified squamous keratinized epithelium or non-keratinized epithelium. For example, penetration enhancers may influence the structure of the tight junctions or lipid vesicles in the non-keratinized epithelium. Suitable permeation enhancers according to the invention are C6 to C12 fatty acids and salts thereof and bile acids and salts thereof. In this context, the term "fatty acid" is understood to mean an aliphatic compound comprising at least one carboxylic acid group (COOH). The fatty acid may be saturated or unsaturated, linear or branched, and cyclic or acyclic. For example, the fatty acid may be caproic acid, heptanoic acid, caprylic acid, nonanoic acid, capric acid, undecanoic acid or lauric acid. The term "bile acid" as used herein includes any steroid acids (and/or the carboxylate anion thereof), and salts thereof, found in the bile of an animal (e.g., a human), including, by way of non-limiting example, cholic acid, cholate, deoxycholic acid, deoxycholate,
hyodeoxycholic acid, hyodeoxycholate, glycocholic acid, glycocholate, taurocholic acid, taurocholate, chenodeoxycholic acid, chenodeoxycholate, lithocholic acid, lithocholate, and the like.

Within the meaning of the invention, the term "salt" is used in the broadest sense and preferably is a pharmaceutically acceptable salt. For example, the term "salt" includes alkali salts of C6 to C12 fatty acids such as sodium caprate as well as alkali salts of bile acids such as sodium glycocholate.

Within the meaning of this invention, the term "polymer" refers to any substance consisting of so-called repeating units obtained by polymerizing one or more monomers, and includes homopolymers which consist of one type of monomer and copolymers which consist of two or more types of monomers. Polymers may be of any architecture such as linear polymers, star polymer, comb polymers, brush polymers, of any monomer arrangements in case of copolymers, e.g. alternating, statistical, block copolymers, or graft polymers. The minimum molecular weight varies depending on the polymer type and is known to the skilled person. Polymers may e.g. have a molecular weight above 2,000, preferably above 5,000 and more preferably above 10,000 g/mol. Correspondingly, compounds with a molecular weight below 2,000, preferably below 5,000 or more preferably below 10,000 g/mol are usually referred to as oligomers.

The oromucosal dosage forms such as oromucosal therapeutic systems comprising the composition according to the present invention can be characterized by certain parameters as measured in an *in vitro* permeation test.

The *in vitro* permeation test can be performed with human or animal mucosa or with an oral mucosal model (human organotypic oral epithelial tissue cultures), e.g., with phosphate buffer pH 7.4 as receptor medium (37 °C) with or without addition of a maximum of 20 vol-% organic solvent. Where not otherwise indicated, the *in vitro* permeation test is performed with human organotypic oral epithelial tissue cultures, and with phosphate buffer pH 7.4 (with 0.1 % (w/v%) sodium azide as antibacteriological agent) as receptor medium (37 °C). Human organotypic oral epithelial tissue cultures are commercially available under the brand name EpiOral^{™} (ORL-200), developed by MatTek. The tissue consists of normal, human-derived epithelial cells. The cells have been cultured to form multilayered, highly differentiated models of the human buccal (EpiOral) phenotypes. The EpiOral tissue model exhibits *in vivo*-like morphological and growth characteristics which are uniform and highly reproducible. Morphologically, the tissue model closely parallels native human tissue, thus providing a useful *in-vitro* means to assess *in-vivo* permeability of pharmaceutical formulations across buccal mucosa.

The amount of active permeated into the receptor medium is determined in regular intervals using an HPLC method with a UV photometric detector by taking a sample volume. The measured amount of active permeated relates to the amount permeated between the two last sampling points and not the total amount permeated so far.

Thus, within the meaning of this invention, the parameter "permeated amount" is provided in µg/cm² and relates to the amount of active permeated in a sample interval at certain elapsed time per area of release. E.g., in an *in vitro* permeation test as described above, wherein the amount of active permeated into the receptor medium has been e.g. measured at hours 1, 2, 3, (4,) 5 and optionally 8, the "permeated amount" of active can be given e.g. for the sample interval from hour 2 to hour 3 and corresponds to the measurement at hour 3.

The permeated amount can also be given as a "cumulative permeated amount", corresponding to the cumulated amount of active permeated at a certain point in time. E.g., in an *in vitro* permeation test as described above, wherein the amount of active permeated into the receptor medium has been e.g. measured at hours 1, 2, 3, (4,) 5 and optionally 8, the "cumulative permeated amount" of active at hour 3 corresponds to the sum of the permeated amounts from hour 0 to hour 1, hour 1 to hour 2, and hour 2 to hour 3.

A "mucosa permeation rate" for a certain sample interval at certain elapsed time, provided in µg/(cm² h), can be calculated from the permeated amount in said sample interval as measured by *in vitro* permeation test as described above in µg/cm², divided by the hours of said sample interval. E.g. the mucosa permeation rate in an *in vitro* permeation test as described above, wherein the amount of active permeated into the receptor medium has been e.g. measured at hours 1, 2, 3, (4,) 5 and optionally 8, the "mucosa permeation rate" at hour 3 is calculated as the permeated amount in the sample interval from hour 2 to hour 3 divided by 1 hour.

A "cumulative mucosa permeation rate" can be calculated from the respective cumulative permeated amount by dividing the cumulative permeated amount by the elapsed time. E.g. in an *in vitro* permeation test as described above, wherein the amount of active permeated into the receptor medium has been e.g. measured at hours 1, 2, 3, (4,) 5 and optionally 8, the "cumulative mucosa permeation rate" at hour 3 is calculated as the cumulative permeated amount for hour 3 (see above) divided by 3 hours.

Within the meaning of this invention, the above parameters permeated amount and mucosa permeation rate as well as cumulative permeated amount and cumulative mucosa permeation rate refer to mean values calculated from 3 *in vitro* permeation test experiments. Where not otherwise indicated, the standard deviation (SD) of these mean values refer to a corrected sample standard deviation calculated using the formula: $SD = \sqrt{\frac{1}{n - 1} {\sum }_{i = 1}^{n} {\left({x}_{i}-\overline{x}\right)}^{2}}$ wherein n is the sample size, {*x₁, x₂,* ... *xₙ*} are the observed values and *x̅* is the mean value of the observed values.

Within the meaning of the invention, the term "total amount permeated" refers to the entire amount of active permeated within the period of application. In this context, "the period of application" is understood to mean the time starting with administering the oromucosal therapeutic system and ending with complete dissolution of the active agent-containing layer.

Within the meaning of this invention, the term "administration" refers to the application of the dosage form, i.e. the oromucosal therapeutic system, to the oral mucosa of the patient, which is then maintained on the mucosa until the active agent-containing layer is dissolved.

The skin irritation potential of the penetration enhancers according to the invention can be predicted using an *in vitro* cell viability assay. The test consists of an exposure of an oral mucosal model (human organotypic oral epithelial tissue cultures, EpiOral^{™} manufactured by MatTek) to a test substance comprising the penetration enhancers, followed by a cell viability test in accordance with the MTT effective time-50 ET-50) protocol (MatTek). In the test, cell viability is measured by dehydrogenase conversion of MTT [(3-4,5-dimethyl thiazole 2-yl) 2,5-diphenyltetrazoliumbromide], present in cell mitochondria, into a blue formazan salt that is quantitatively measured by optical density measurement (providing the absorbance, also called optical density, as OD) after extraction from tissues. The reduction of the viability of tissues exposed to chemicals in comparison to negative controls (treated with artificial saliva) is used to predict the skin irritation potential. For each individual tissue treated with a test substance (TS), the positive control (PC) and the negative control (NC), the individual relative tissue viability is calculated according to the following formulas: $Relative viability TS \left(%\right) = \left[{OD}_{TS}/Mean of {OD}_{NC}\right] \times 100$ $Relative viability NC \left(%\right) = \left[{OD}_{NC}/mean of {OD}_{NC}\right] \times 100$ $Relative viability PC \left(%\right) = \left[{OD}_{PC}/mean of {OD}_{NC}\right] \times 100 .$

Within the meaning of this invention, the term "room temperature" refers to the unmodified temperature found indoors in the laboratory where the experiments are conducted and usually lies within 15 to 35 °C, or about 18 to 25 °C.

Within the meaning of this invention, the term "patient" refers to a subject who has presented a clinical manifestation of a particular symptom or symptoms suggesting the need for treatment, who is treated preventatively or prophylactically for a condition, or who has been diagnosed with a condition to be treated. The condition may be diabetes and/or obesity.

Within the meaning of this invention, the term "coating composition" refers to a composition comprising all components of the drug-containing layer in a solvent, which may be coated onto the backing layer or release liner to form the drug-containing layer upon drying.

Within the meaning of this invention, the term "dissolve" in the context of the preparation of the coating composition, e.g. dissolving components of the coating composition such as the active agent, refers to the process of obtaining a solution, which is clear and does not contain any particles, as visible to the naked eye.

Within the meaning of this invention, the term "solvent" refers to any liquid substance, such as water.

Within the meaning of this invention, and unless otherwise specified, the term "about" refers to an amount that is ± 10 % of the disclosed amount. In some embodiments, the term "about" refers to an amount that is ± 5 % of the disclosed amount. In some embodiments, the term "about" refers to an amount that is ± 2 % of the disclosed amount.

### BRIEF DESCRIPTION OF THE DRAWINGS

Fig. 1a depicts the cumulative permeated amount of semaglutide across reconstructed human epithelial tissue after application of solutions according to Pre-Examples 1a, 1c and 1d for hours 0 to 8.
Fig. 1b depicts the cumulative permeated amount of semaglutide across reconstructed human epithelial tissue after application of solutions according to Pre-Examples 1a, 1e and 1f for hours 0 to 8.
Fig. 1c depicts the cumulative permeated amount of semaglutide across reconstructed human epithelial tissue after application of solutions according to Pre-Examples 1a, 1d, 1f and 1g for hours 0 to 8.
Fig. 1d depicts the utilization of semaglutide across reconstructed human epithelial tissue after application of solutions according to Pre-Examples 1a, 1c and 1d at hour 5.
Fig. 1e depicts the utilization of semaglutide across reconstructed human epithelial tissue after application of solutions according to Pre-Examples 1a, 1e and 1f at hour 5.
Fig. 1f depicts the utilization of semaglutide across reconstructed human epithelial tissue after application of solutions according to Pre-Examples 1a, 1d, 1f and 1g at hour 5.
Fig. 1g depicts the cumulative permeated amount of buserelin across porcine mucosa after application of solutions according to Pre-Examples 1h, 1i and 1j for hours 0 to 6.
Fig. 1h depicts the cumulative permeated amount of buserelin across reconstructed human epithelial tissue after application of solutions according to Pre-Examples 1k and 1l for hours 0 to 5.
Fig. 1i depicts the cumulative permeated amount of sumatriptan across porcine mucosa after application of solutions according to Pre-Examples 1m, 1n and 1o for hours 0 to 6.
Fig. 2a depicts the cumulative permeated amount of semaglutide across reconstructed human epithelial tissue after application of a solution according to Pre-Example 2a as well as an oromucosal therapeutic system according to Example 2d for hours 0 to 5.

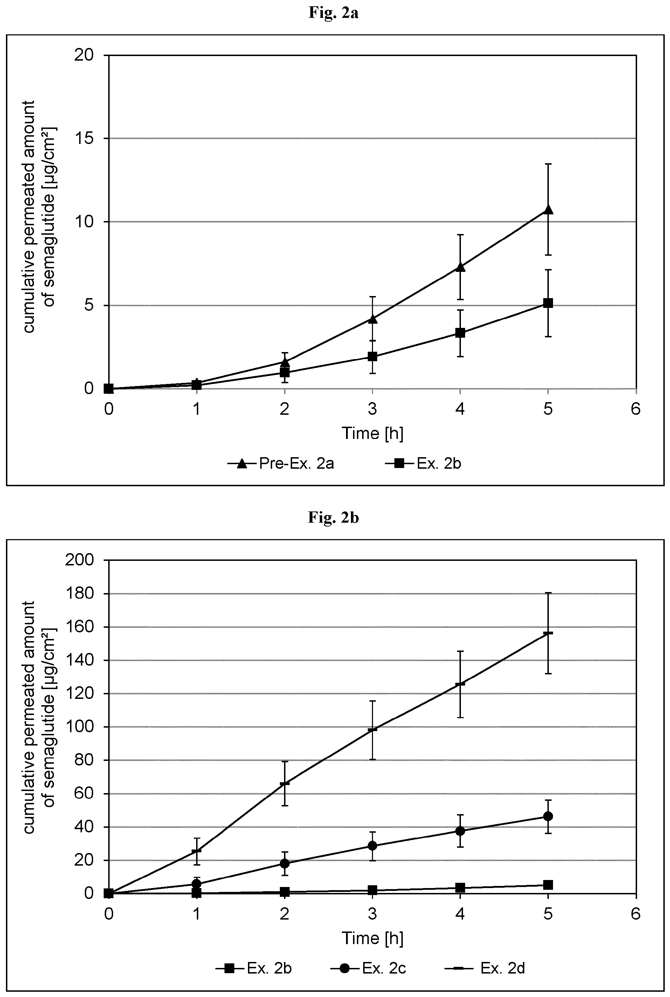

Fig. 2a depicts the cumulative permeated amount of semaglutide across reconstructed human epithelial tissue after application of a solution according to Pre-Example 2a as well as an oromucosal therapeutic system according to Example 2b for hours 0 to 5.
Fig. 2b depicts the cumulative permeated amount of semaglutide across reconstructed human epithelial tissue after application of oromucosal therapeutic systems according to Examples 2b to 2d for hours 0 to 5.

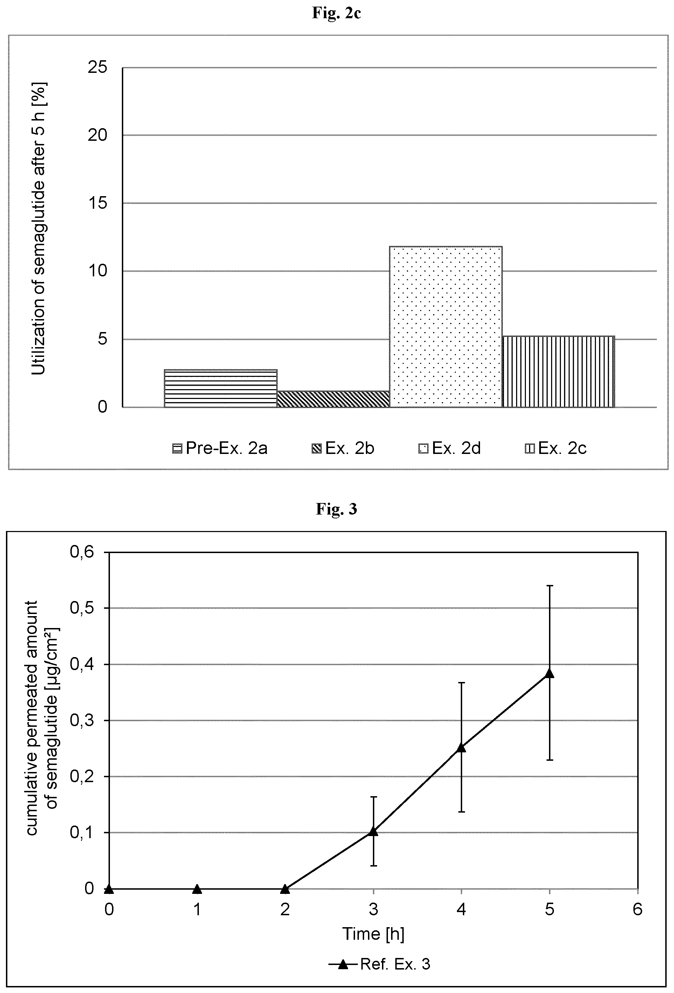

Fig. 2c depicts the utilization of semaglutide across reconstructed human epithelial tissue after application of a solution according to Pre-Example 2a as well as oromucosal therapeutic systems according to Examples 2b to 2d at hour 5.
Fig. 3 depicts the cumulative permeated amount of semaglutide across reconstructed human epithelial tissue after application of an oromucosal therapeutic system according to Reference Example 3 for hours 0 to 5.

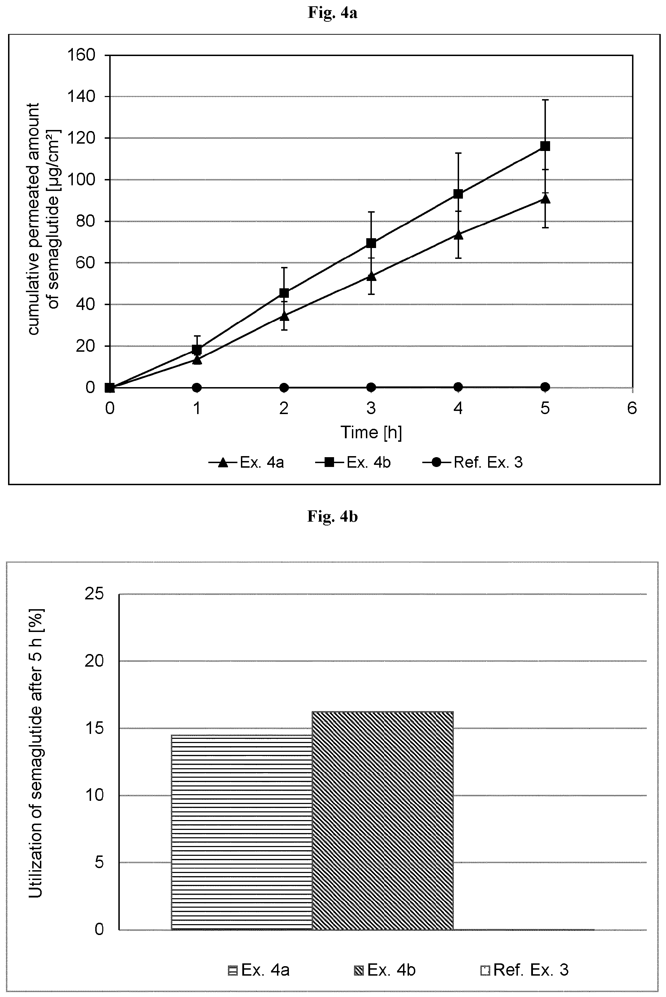

Fig. 4a depicts the cumulative permeated amount of semaglutide across reconstructed human epithelial tissue after application of oromucosal therapeutic systems according to Examples 4a, 4b and Reference Example 3 for hours 0 to 5.
Fig. 4b depicts the utilization of semaglutide across reconstructed human epithelial tissue after application of oromucosal therapeutic systems according to Examples 4a, 4b and Reference Example 3 at hour 5.

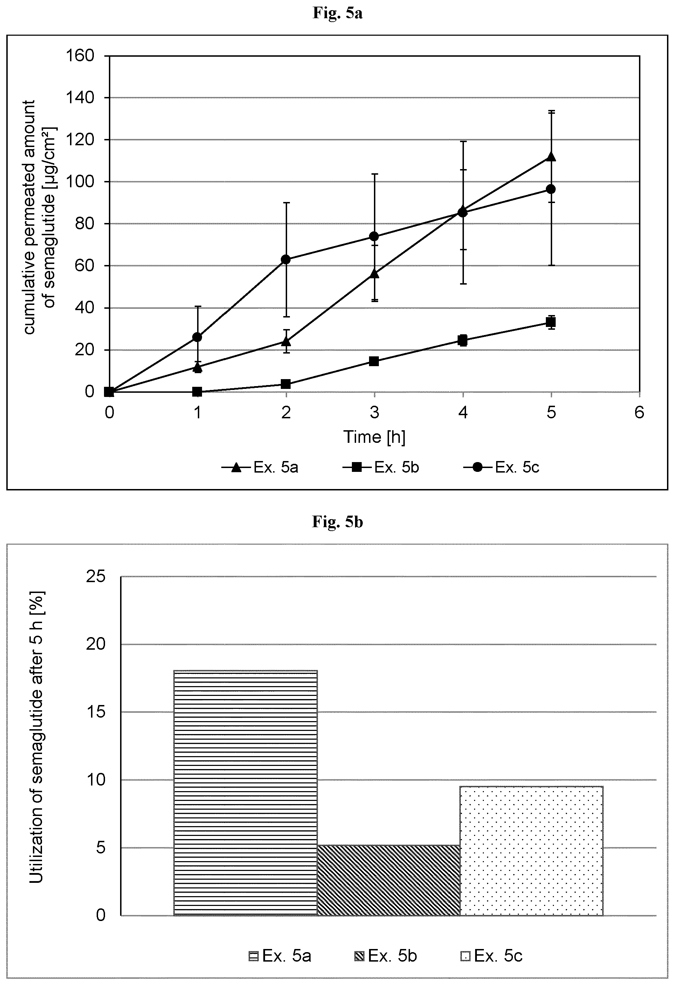

Fig. 5a depicts the cumulative permeated amount of semaglutide across reconstructed human epithelial tissue after application of oromucosal therapeutic systems according to Examples 5a, 5b and 5c for hours 0 to 5.
Fig. 5b depicts the utilization of semaglutide across reconstructed human epithelial tissue after application of oromucosal therapeutic systems according to Examples 5a, 5b and 5c at hour 5.

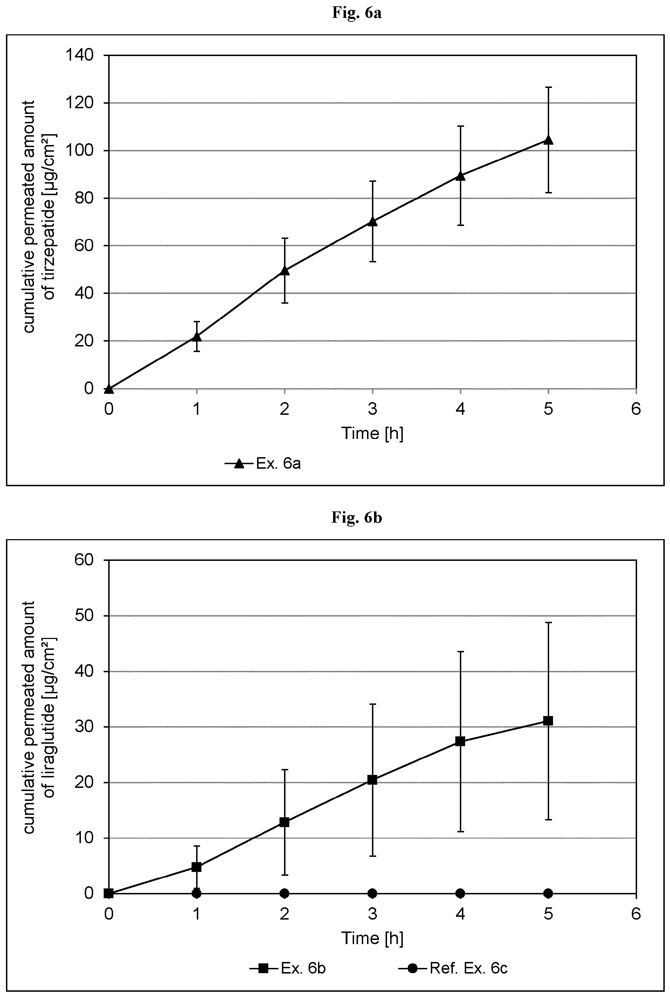

Fig. 6a depicts the cumulative permeated amount of tirzepatide across reconstructed human epithelial tissue after application of an oromucosal therapeutic system according to Example 6a for hours 0 to 5.
Fig. 6b depicts the cumulative permeated amount of liraglutide across reconstructed human epithelial tissue after application of oromucosal therapeutic systems according to Example 6b and Reference Example 6c for hours 0 to 5.

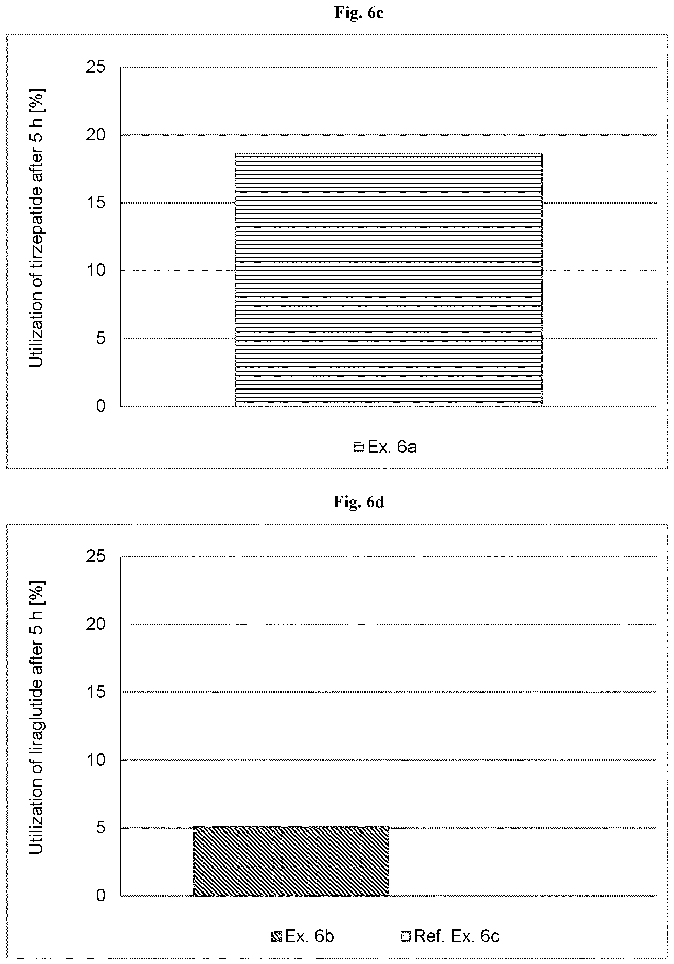

Fig. 6c depicts the utilization of tirzepatide across reconstructed human epithelial tissue after application of an oromucosal therapeutic system according to Example 6a at hour 5.
Fig. 6d depicts the utilization of liraglutide across reconstructed human epithelial tissue after application of oromucosal therapeutic systems according to Example 6b and Reference Example 6c at hour 5.

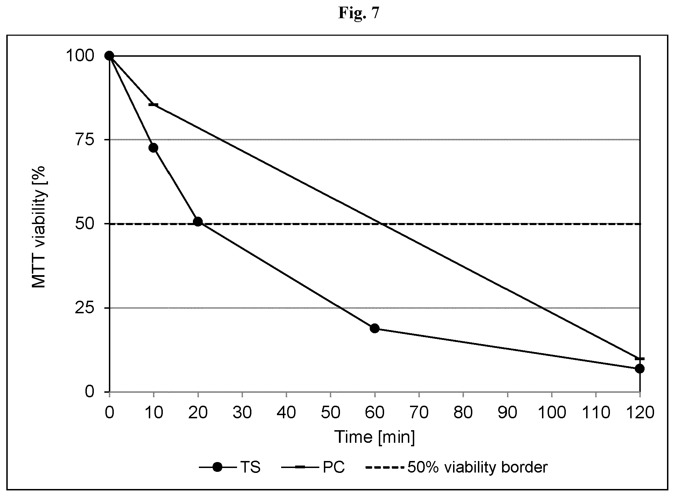

Fig. 7 depicts the MTT cell viability of reconstructed human epithelial tissue after application of a test substance (TS) as compared to a positive control (PC) for minutes 0 to 120 as measured according to the skin irritation potential experiment 7.

### DETAILED DESCRIPTION

### COMPOSITION FOR OROMUCOSAL ADMINISTRATION

The present invention is related to a composition for the oromucosal administration of an active agent comprising A) the active agent, and B) a combination of permeation enhancers comprising a first permeation enhancer and a second permeation enhancer. The first permeation enhancer is selected from the group consisting of C6 to C12 fatty acids and salts thereof, while the second permeation enhancer selected from the group consisting of bile acids and salts thereof.

Thus, the composition for the oromucosal administration of an active agent of the present invention comprises
A) the active agent, and
B) a combination of permeation enhancers comprising
   i) a first permeation enhancer selected from the group consisting of C6 to C12 fatty acids and salts thereof; and
   ii) a second permeation enhancer selected from the group consisting of bile acids and salts thereof.

The composition can be in any form suitable for oromucosal administration, which means that e.g. any solid form which is too large or has an inappropriate shape for application in the oral cavity is not envisaged by the present invention. In certain embodiments, the composition is a liquid composition or is a solid composition.

In certain embodiments, the composition is a solid composition in the form of a powder, granules, or a monolithic structure. In some of these embodiments, the monolithic structure is a tablet or a film or a part of a tablet or a film, e.g., a tablet layer, a tablet core, a tablet shell or a film layer. As will be outlined in further detail below, the composition can be used as the final dosage form, in case the composition is already in appropriate form, or may have to be further formulated or processed to provide the final dosage form. E.g., the composition in the form of a tablet or a film may represent the final dosage form, while a power could be processed by adding further excipients to provide a paste which is easier to apply in the oral cavity than a powder.

### COMBINATION OF PERMEATION ENHANCERS

As outlined above, the composition of the present invention comprises a combination of permeation enhancers comprising a first permeation enhancer and a second permeation enhancer. The permeation enhancers as defined herein increase the amount of active agent released to the systemic circulation. Without wishing to be bound by theory, it is believed that the permeation enhancers may *inter alia* affect the structure or the fluidization of the transcellular membrane to increase the permeability, and may also act as a solubilizer for the active agent.

Advantageously, the combination of the first permeation enhancer and the second permeation enhancer achieves a synergistic effect, resulting in an excellent and improved active delivery across the oral mucosa, which exceeds what would have been expected from the increase in active release obtained for each of the penetration enhancers independently.

The first permeation enhancer according to the present invention is selected from the group consisting of C6 to C12 fatty acids and salts thereof. In certain embodiments, the first permeation enhancer is selected from the group consisting of caproic acid, heptanoic acid, caprylic acid, nonanoic acid, capric acid, undecanoic acid, lauric acid and salts thereof. In certain specific embodiments, the first permeation enhancer is selected from the group consisting of caproic acid, caprylic acid, capric acid, lauric acid and salts thereof. In particular, according to certain specific embodiments, the first permeation enhancer is a salt, such as an alkali salt, of a fatty acid selected from the group consisting of caproic acid, caprylic acid, capric acid and lauric acid, such as a sodium salt or potassium salt of a fatty acid selected from the group consisting of caproic acid, caprylic acid, capric acid or lauric acid.

Thus, according to certain specific embodiments, the first permeation enhancer is selected from the group consisting of sodium caproate, sodium caprylate, sodium caprate, sodium laurate, potassium caproate, potassium caprylate, potassium caprate, and potassium laurate. In certain more specific embodiments, the first permeation enhancer is selected from the group consisting of sodium caprate and potassium caprate.

In particular, according to further specific embodiments, the first permeation enhancer is sodium caprate.

The second permeation enhancer according to the present invention is selected from the group consisting of bile acids and salts thereof. In certain embodiments, the second permeation enhancer is selected from the group consisting of cholic acid, taurocholic acid, glycocholic acid, deoxycholic acid, taurodeoxycholic acid, glycodeoxycholic acid, chenodeoxycholic acid, taurochenodeoxycholic acid, glycochenodeoxycholic acid, and salts thereof. In particular, according to certain specific embodiments, the second permeation enhancer is a salt, such as an alkali salt, of a bile acid selected from the group consisting of cholic acid, taurocholic acid, glycocholic acid, deoxycholic acid, taurodeoxycholic acid, glycodeoxycholic acid, chenodeoxycholic acid, taurochenodeoxycholic acid, and glycochenodeoxycholic acid, such as a sodium salt of a bile acid selected from the group consisting of cholic acid, taurocholic acid, glycocholic acid, deoxycholic acid, taurodeoxycholic acid, glycodeoxycholic acid, chenodeoxycholic acid, taurochenodeoxycholic acid, and glycochenodeoxycholic acid.

Thus, according to certain specific embodiments, the second permeation enhancer is selected from the group consisting of sodium cholate, sodium taurocholate, sodium glycocholate, sodium deoxycholate, sodium taurodeoxycholate, sodium glycodeoxycholate, sodium ursodeoxycholate, sodium chenodeoxycholate, sodium taurochenodeoxycholate, sodium glycochenodeoxycholate, sodium cholylsarcosine and sodium N-methyl taurocholate. In certain more specific embodiments, the second permeation enhancer is selected from the group consisting of sodium glycocholate and sodium taurotaurodeoxycholate.

In particular, according to further specific embodiments, the second permeation enhancer is sodium glycocholate.

According to certain embodiments, if the first permeation enhancer is sodium caprate, the second permeation enhancer is not sodium chenodeoxycholate. In particular, if the first permeation enhancer is sodium caprate, the second permeation enhancer may be selected from the group consisting of sodium cholate, sodium taurocholate, sodium glycocholate, sodium deoxycholate, sodium taurodeoxycholate, sodium glycodeoxycholate, sodium ursodeoxycholate, sodium taurochenodeoxycholate, sodium glycochenodeoxycholate, sodium cholylsarcosine and sodium N-methyl taurocholate.

According to certain other embodiments, if the first permeation enhancer is sodium caprate, the second permeation enhancer is not sodium deoxycholate. In particular, if the first permeation enhancer is sodium caprate, the second permeation enhancer may be selected from the group consisting of sodium cholate, sodium taurocholate, sodium glycocholate, sodium chenodeoxycholate, sodium taurodeoxycholate, sodium glycodeoxycholate, sodium ursodeoxycholate, sodium taurochenodeoxycholate, sodium glycochenodeoxycholate, sodium cholylsarcosine and sodium N-methyl taurocholate.

According to yet certain other embodiments, if the first permeation enhancer is sodium caprate, the second permeation enhancer is neither sodium chenodeoxycholate nor sodium deoxycholate. In particular, if the first permeation enhancer is sodium caprate, the second permeation enhancer may be selected from the group consisting of sodium cholate, sodium taurocholate, sodium glycocholate, sodium taurodeoxycholate, sodium glycodeoxycholate, sodium ursodeoxycholate, sodium taurochenodeoxycholate, sodium glycochenodeoxycholate, sodium cholylsarcosine and sodium N-methyl taurocholate.

According to certain specific embodiments,
i) the first permeation enhancer is a salt of a fatty acid selected from the group consisting of caproic acid, caprylic acid, capric acid and lauric acid; and
ii) the second permeation enhancer is a salt of a bile acid selected from the group consisting of cholic acid, taurocholic acid, glycocholic acid, deoxycholic acid, taurodeoxycholic acid, glycodeoxycholic acid, ursodeoxycholic acid, chenodeoxycholic acid, taurochenodeoxycholic acid, glycochenodeoxycholic acid, sarcocholic acid and N-methyl taurocholic acid,
or
i) the first permeation enhancer is selected from the group consisting of sodium caproate, sodium caprylate, sodium caprate, sodium laurate, potassium caproate, potassium caprylate, potassium caprate, and potassium laurate; and
ii) the second permeation enhancer is selected from the group consisting of sodium cholate, sodium taurocholate, sodium glycocholate, sodium deoxycholate, sodium taurodeoxycholate, sodium glycodeoxycholate, sodium ursodeoxycholate, sodium chenodeoxycholate, sodium taurochenodeoxycholate, sodium glycochenodeoxycholate, sodium cholylsarcosine and sodium N-methyl taurocholate,
or
i) the first permeation enhancer is selected from the group consisting of sodium caprate and potassium caprate; and
ii) the second permeation enhancer is selected from the group consisting of sodium glycocholate and sodium taurodeoxycholate.

According to certain particular embodiments,
i) the first permeation enhancer is sodium caprate; and
ii) the second permeation enhancer sodium glycocholate.

Thus, according to certain particular embodiments, the composition for the oromucosal administration of an active agent of the present invention comprises
A) the active agent, and
B) a combination of permeation enhancers comprising
   i) sodium caprate as first permeation enhancer; and
   ii) sodium glycocholate as second permeation enhancer.

In terms of the amount of permeation enhancer, without wishing to be bound by theory, it is believed that increasing the amount of first and/or second permeation enhancer (and thus enlarging the weight ratio of first permeation enhancer or second permeation enhancer, respectively, to active agent), also increases the amount of active agent released from the transmucosal therapeutic system according to the invention. In other words, a certain amount of enhancer is needed in order to ensure a sufficient degree of increase in active delivery. On the other hand, if the amount of first and/or second permeation enhancer is too high, the composition will be difficult to formulate, in particular since a larger amount of enhancer will result in increased size or volume of the medicament. In addition, a large amount of enhancer might lead to bad taste which is difficult to mask as well as to potential irritating sensations at the mucosa or otherwise in the oral cavity.

Thus, according to certain embodiments, the weight ratio of first or second permeation enhancer to active agent is at least 0.3:1, at least 0.5:1, at least 1:1, or at least 2:1.

In certain embodiments, the weight ratio of first permeation enhancer to active agent is at least 0.3:1, at least 1:1, at least 2:1 or at least 3:1. Also, according to certain embodiments, the weight ratio of first permeation enhancer to active agent is 100:1 or less, 70:1 or less, 50:1 or less, or 40:1 or less, such as 30:1 or less, 10:1 or less, or 5:1 or less. In particular, according to certain embodiments, the weight ratio of first permeation enhancer to active agent is from 0.3:1 to 100:1, from 1:1 to 70:1, from 2:1 to 50:1, or from 3:1 to 40:1. Moreover, according to certain specific embodiments, the weight ratio of first permeation enhancer to active agent is from 2:1 to 10:1, or from 3:1 to 5:1.

In these or other embodiments, the weight ratio of second permeation enhancer to active agent is at least 1.5:1, at least 3:1, at least 10:1, or at least 20:1. Also, in certain embodiments, the weight ratio of second permeation enhancer to active agent is 100:1 or less, 70:1 or less, 50:1 or less, or 40:1 or less, such as 30:1 or less, 10:1 or less, or 5:1 or less. In particular, according to certain embodiments, the weight ratio of second permeation enhancer to active agent is from 1.5:1 to 100:1, from 3:1 to 70:1, from 10:1 to 50:1, or from 20:1 to 40:1. Moreover, according to certain specific embodiments, the weight ratio of second permeation enhancer to active agent is from 2:1 to 10:1, or from 3:1 to 5:1.

In certain particular embodiments, the weight ratios of first permeation enhancer to active agent and of second permeation enhancer to active agent are the same. In particular, according to certain particular embodiments, the weight ratios of first permeation enhancer to active agent and of second permeation enhancer to active agent are from 2:1 to 10:1, or from 3:1 to 5:1.

Accordingly, the weight ratio of first permeation enhancer to second permeation enhancer to active agent is at least 2:2:1, or 10:10:1 or less. In certain particular embodiments, the weight ratio of first permeation enhancer to second permeation enhancer to active agent is about 3:3:1, or about 4:4:1, or about 5:5:1, or about 10:10:1.

In certain embodiments, the weight ratio of first permeation enhancer to second permeation enhancer is at least 1:2, or at least 1:1. Also, in certain embodiments, the weight ratio of first permeation enhancer to second permeation enhancer is 10:1 or less or 6:1 or less, such as 3:1 or less, or 2:1 or less. In particular, according to certain embodiments, the weight ratio of first permeation enhancer to second permeation enhancer is from 1:2 to 10:1, or from 1:1 to 6:1. Moreover, according to certain specific embodiments, the weight ratio of first permeation enhancer to second permeation enhancer is about 1:1.

### THE ACTIVE AGENT

As outlined above, the composition according to the present invention comprises an active agent. In accordance with the invention, the composition according to the present invention preferably comprises an active agent in a therapeutically effective amount.

The combination of penetration enhancers of the present invention is believed to be beneficial for any kind of active agent, and the effect of improving active delivery to be available independent of the type of active agent. Thus, in certain embodiments, the active agent is selected from the group consisting of hormone analogues, opioids, antihistamines, non-steroidal anti-inflammatory agents, anti-emetics, anti-epileptics, vasodilators, anti-tussive agents and expectorants, anti-asthmatics, antacids, anti-spasmodics, antidiabetics, diuretics, anti-hypotensives, antihypertensives, bronchodilators, steroids, antibiotics, antihemorrhoidals, hypnotics, psychotropics, antidiarrheals, mucolytics, sedatives, decongestants, laxatives, vitamins, anti-multiple sclerosis, anti-parkinson, anti-alzheimer, analgesics, stimulants and cannabinoids.

On the other hand, it is understood that the present invention will be most useful for formulating such actives which otherwise would not be available for the oromucosal route, e.g., because of insufficient permeation. Hydrophilic or large active agents typically do not permeate well across mucosa.

Therefore, in certain other embodiments, the active agent has a solubility in water of more than 0.1 mg/mL, more than 0.5 mg/mL, or more than 1 mg/mL. In certain embodiments, the solubility in water is 2 g/mL or less, 1 g/mL or less, or 0.5 g/mL or less. In such embodiments, the solubility in water may be more than 0.1 mg/mL and 2 g/mL or less, more than 0.5 mg/mL and 1 g/mL or less, or more than 1 mg/mL and 0.5 g/mL or less. In yet other embodiments, the active agent is selected from molecules with a molecular weight of at least 50 g/mol, at least 500 g/mol, at least 1,000 g/mol, or at least 3,000 g/mol, or the active agent is selected from molecules with a molecular weight of less than 100,000 g/mol, less than 20,000 g/mol, or less than 5,000 g/mol. In such other embodiments, the active agent can also be selected from molecules with a molecular weight of 50 to less than 500 g/mol, of 500 to less than 1,000 g/mol, of 1,000 to less than 3,000 g/mol, of 3,000 to 10,000 g/mol, of 10,000 to 20,000 g/mol or of 20,000 to 100,000 g/mol. In such other embodiments, the active agent can also be selected from molecules with a molecular weight of 50 to 100,000 g/mol or less, of 500 to 20,000 g/mol or less, of 1,000 to 10,000 g/mol or less, or of 3,000 to 5,000 g/mol or less.

In certain embodiments, the active agent is a hormone analogue, and in particular an LHRH agonist. Exemplary LHRH agonists comprise buserelin (MW = 1239 g/mol), nafarelin (MW = 1323 g/mol), leuprolide (MW = 1209 g/mol), goserelin (MW = 1269 g/mol) and triptorelin (MW = 1311 g/mol).

In certain embodiments, the active agent is an antidiabetic, and in particular a GLP-1 receptor agonist. The GLP-1 receptor agonist used in the composition according to the present invention is not particularly limited as long as it is suitable for oromucosal delivery. Accordingly, the GLP-1 receptor agonist can be selected from peptides or peptide conjugates exhibiting at least partial sequence identity, such as at least 20% sequence identity, at least 50% sequence identity, at least 75% sequence identity or at least 90% sequence identity, with endogenous GLP-1(7-37).

Surprisingly, it has been found that GLP-1 receptor agonists having a molecular weight of 1,000 g/mol or more, such as 3,000 g/mol or more, can be delivered oromucosally in sufficient amounts when using the specific combination of permeation enhancers of the present invention. Thus, according to certain embodiments, the GLP-1 receptor agonist is selected from peptides or peptide conjugates having a molecular weight of 1,000 g/mol or more, 3,000 g/mol or more, 3,500 g/mol or more, or 3,750 g/mol or more, or the GLP-1 receptor agonist is selected from peptides or peptide conjugates having a molecular weight of 100,000 g/mol or less, 20,000 g/mol or less, 10,000 g/mol or less, or 5,000 g/mol or less. In particular, the GLP-1 receptor agonist may be selected from peptides or peptide conjugates having a molecular weight of 1,000 g/mol or more and 100,000 g/mol or less, 3,000 g/mol or more and 20,000 g/mol or less, 3,500 g/mol or more and 10,000 g/mol or less, or 3,750 g/mol or more and 5,000 g/mol or less.

In certain embodiments, the GLP-1 receptor agonist is selected from the group consisting of albiglutide, beinaglutide, cotadutide, dulaglutide, efinopegdutide, efocipegtrutide, efpeglenatide, exenatide, liraglutide, lixisenatide, pemvidutide, retatrutide, semaglutide, survodutide, taspoglutide, and tirzepatide. In certain specific embodiments, the GLP-1 receptor agonist is selected from the group consisting of liraglutide, semaglutide and tirzepatide. In certain more specific embodiments, the GLP-1 receptor agonist is semaglutide.

### DOSAGE FORMS COMPRISING THE COMPOSITION

The composition of the present invention is used for oromucosal administration of the active agent contained in the composition. To this end, the composition can be directly used as a dosage form, if already in appropriate form. E.g., if the composition is a solid composition in the form of a monolithic structure such as a tablet that can be sucked, such tablet can be directly administered, i.e., the composition in this case is already the final dosage form.

The composition can be also further formulated or processed by adding further excipients and/or further constituents of the final dosage form that is administered to a patient. As outlined above, for example, a powder could be processed by adding further excipients to provide a paste. As another example, if the composition is in the form of an oromucosal film, the film can be used to form and be part of an oromucosal therapeutic system further comprising, e.g., a detachable protective layer on which the oromucosal film is located, and/or a backing layer on top of the oromucosal film.

Thus, in certain embodiments, the present invention relates to an oromucosal dosage form such as an oromucosal therapeutic system comprising the composition for the oromucosal administration of an active agent as described above. In certain embodiments, such an oromucosal delivery system is in the form of a tablet or a film.

In certain further specific embodiments, the oromucosal therapeutic system is in the form of a film and comprises the composition as described above as an active agent-containing layer. In certain of such embodiments, the oromucosal therapeutic system is in the form of a film for the oromucosal administration of an active agent and comprises a mucoadhesive layer structure, said mucoadhesive layer structure comprising the composition as described above as an active agent-containing layer, the active agent-containing layer comprising
A) the active agent, and
B) the combination of permeation enhancers as described above.

In certain of such embodiments, the active agent-containing layer may also further comprise a dissolvable film-forming agent. In yet further of such embodiments, the active agent is a GLP-1 receptor agonist or an LHRH agonist and in particular is selected from the group consisting of albiglutide, beinaglutide, dulaglutide, efinopegdutide, efocipegtrutide, efpeglenatide, exenatide, liraglutide, lixisenatide, pemvidutide, retatrutide, semaglutide, survodutide, taspoglutide, and tirzepatide, or is selected from the group consisting of buserelin, nafarelin, leuprolide, goserelin and triptorelin, or is semaglutide or is buserelin.

In these embodiments, the oromucosal therapeutic system can be designed as appropriate and known to the skilled person. E.g., a dissolvable film-forming agent can be chosen as needed in view of the overall properties of the oromucosal therapeutic system such as mucoadhesivity, disintegration time and active release profile.

Such a dissolvable film-forming agent should be able not only to provide sufficient cohesion to the active agent-containing layer, but preferably provides a film that is not tacky in dry state so that the patient is able to touch and manipulate the active agent-containing layer, e.g. apply it to the oral mucosa, without the same adhering to the fingers. In addition, since the dissolvable film-forming agent is the primary control over the dissolution behavior of the active agent-containing layer which needs to be neither too fast nor too slow, the dissolvable film-forming agent is preferably soluble, dispersible or otherwise disintegrable in aqueous media, specifically in saliva, or, simplified, in water. Thus, selecting the film-forming agent is not a simple task.

In certain embodiments, the dissolvable film-forming agent is selected from the group consisting of polymers such as polyvinylpyrrolidone, methyl cellulose, hydroxyethyl cellulose, hydroxypropyl cellulose, hydroxypropyl methyl cellulose, carboxymethyl cellulose sodium, polyethylene glycol- polyvinyl acetate- and polyvinylcaprolactame-based graft copolymers, polyvinyl alcohol, polyvinyl alcohol-polyethylene glycol copolymers, polyvinylpyrrolidone-polyvinylacetate copolymers, polyethylene oxides, polyethylene glycols, and any mixtures thereof. In particular, according to certain embodiments, the dissolvable film-forming agent is selected from the group consisting of polymers such as methyl cellulose, hydroxyethyl cellulose, hydroxypropyl cellulose, hydroxypropyl methyl cellulose, carboxymethyl cellulose sodium, and any mixtures thereof.

According to certain specific embodiments, the dissolvable film-forming agent comprises a hydroxypropyl methyl cellulose or a mixture of hydroxypropyl methyl celluloses. In particular, according to certain specific embodiments, the dissolvable film-forming agent comprises a mixture of at least two hydroxypropyl methyl celluloses, such as a mixture of a first hydroxypropyl methyl cellulose, a second hydroxypropyl methyl cellulose, and optionally a third and further hydroxypropyl methyl celluloses.

The content of active agent will be adjusted so that the overall size, thickness and active release of the oromucosal therapeutic system is appropriate for achieving the therapeutic effect while at the same time not causing any discomfort to the patient upon administration such as a foreign body feeling, bad taste or irritation at the administration site.

Likewise, further properties of the oromucosal therapeutic system such as the thickness of the film and the area weight as well as the area of release are adjusted as needed. In general, an oromucosal therapeutic system in the form of a film preferably has a certain degree of thickness, as otherwise it will be difficult to incorporate the required amount of active, and as very thin films are not easy to manufacture, in particular with respect to providing an even thickness. Thus, in certain embodiments, the oromucosal therapeutic system is in the form of a thin film having an area weight of at least 40 g/m², at least 80 g/m², or at least 100 g/m². On the other hand, very thick films will be perceived by the patient as a disturbing object in the oral cavity, and thus are disadvantageous in terms of patient compliance. Thus, in certain embodiments, the oromucosal therapeutic system is in the form of a thin film having an area weight of less than or equal to 500 g/m², less than or equal to 300 g/m², or less than or equal to 250 g/m². In particular, according to certain embodiments, the oromucosal therapeutic system is in the form of a thin film having an area weight of from 40 to 500 g/m², from 80 to 300 g/m², or from 80 to 250 g/m². As concerns the size of the film, a certain minimum size is required in order to ensure that the film does not detach prematurely from the mucosa, and also for being able to include a sufficient amount of active without having to use very thick films. On the other hand, if the area of release is too large, the oromucosal therapeutic system will be huge in size, uncomfortable to apply and to wear, leading to low patient compliance. Considering this, according to certain embodiments, the oromucosal therapeutic system has an area of release of at least 0.1 cm², at least 0.2 cm², or at least 0.5 cm², or has an area of release of less than or equal to 10 cm², less than or equal to 7 cm², or less than or equal to 5 cm², or has an area of release of from 0.1 to 10 cm², from 0.2 to 7 cm², or from 0.5 to 5 cm².

### FURTHER ADDITIVES

In certain embodiments, further excipients are added to the composition, as adequate, e.g., the composition according to the invention may comprise further excipients or additives selected from the group consisting of taste masking agents, sweeteners, flavoring agents, colorants, permeation enhancers, solubilizers, plasticizers, humectants, disintegrants, emulsifiers, antioxidants, stabilizers, buffer reagents, further permeation enhancers and film-forming agents.

In view of the potentially unpleasant olfactory-gustatory sensation caused by the penetration enhancers, substances that are able to mask or modify the same are preferred. Thus, according to certain embodiments, the composition comprises one or more excipients selected from the group consisting of taste masking agents, sweeteners, and flavoring agents.

In certain embodiments, the composition comprises one or more natural or artificial taste masking agents (such as ion-exchange agents) selected from the group consisting of flavor suppressing agents, bitter masking agents, sour masking agents and salty masking agents.

In certain embodiments, the composition comprises one or more natural or artificial sweeteners selected from the group consisting of saccharose, glucose, fructose, sorbitol, mannitol, isomalt, maltitol, lactitol, xylitol, erythritol, sucralose, acesulfame potassium, aspartame, cyclamate, neohesperidine, neotame, steviol glycosides, thaumatin and saccharin sodium.

In certain embodiments, the composition comprises one or more natural or artificial flavoring agents selected from the group consisting of vanillin, methyl salicylate, menthol, manzanate, diacetyl, acetylpropionyl, acetoin, isoamyl acetate, benzaldehyde, cinnamaldehyde, ethyl propionate, methyl anthranilate, limonene, ethyl decadienoate, allyl hexanoate, ethyl maltol, 2,4-dithiapentane, ethylvanillin and eucalyptol as well as flavoring compositions such as peppermint flavor.

In certain embodiments, the composition comprises one or more colorants. Any colorant suitable for use in pharmaceutical / food applications can be included, in particular those admitted for use by the US FDA or by the European Agencies EFSA / EMA. Such colorants may be e.g. selected from the group consisting of titanium dioxide, brilliant blue FCF, indigo carmine, fast green FCF, erythrosine, allura red AC, tartrazine and sunset yellow FCF, curcumin, riboflavin, rivoflavin-5'-phosphate, quinoline yellow, orange yellow S, cochineal, carminic acid, azorubine, carmoisine, amaranth, ponceau 4R, cochineal red A, patent blue V, indigotine, chlorophylls, chlorophyllins, copper complexes of chlorophyll and chlorophyllins, green S, plain caramel, caustic sulphite caramel, ammonia caramel, sulphite ammonia caramel, brilliant black BN, black PN, vegetable carbon, brown HT, carotenes, annatto, bixin, norbixin, paprika extract, capsanthian, capsorubin, lycopene, beta-apo-8'-carotenal, lutein, canthaxanthin, beetroot red, betanin, anthocyanins, calcium carbonate, iron oxides and hydroxides, aluminium, silver, gold and litholrubine BK.

In certain embodiments, the composition may further comprise one or more solubilizers. Suitable solubilizers may be, e.g., selected from the group consisting of ethoxylated sorbitan esterified with fatty acids such as polyoxyethylene sorbitan monolaurate, polyoxyethylene sorbitan monopalmitate, polyoxyethylene sorbitan monostearate and polyoxyethylene sorbitan monooleate (commercially available as Tween 80 or Polysorbate 80), safflower oleosomes, propanediol and polyethoxylated castor oil.

In certain embodiments, the composition may further comprise one or more emulsifiers. Suitable emulsifiers may be, e.g., selected from the group consisting of soy lecithin, sodium phosphates, mono- and diglycerides of fatty acids, sodium stearoyl lactylate, diacetyl tartaric acid esters of mono- and diglycerides, and polyethoxylated hydrogenated castor oil (commercially available as Cremophor RH 40 from BASF).

In certain embodiments, the composition may further comprise one or more plasticizers. Suitable plasticizers may be selected from the group consisting of mono-, di-, oligo- and polysaccharides and derivatives such as sorbitol (commercially available as Sorbidex^{™} from Cargill), polyethylene glycol, triacetin, triethyl citrate, propylene glycol, glycerol and medium chain triglycerides.

### METHOD OF TREATMENT/ MEDICAL USE

In accordance with a specific aspect of the present invention, the composition for the oromucosal administration of an active agent according to the invention, or a dosage form comprising the composition, is for use in a method of treatment, and in particular for use in a method of treating a human patient. In accordance with another aspect, the present invention is related to a method of treatment, wherein the composition for the oromucosal administration of an active agent according to the invention, or a dosage form comprising the composition, is oromucosally administered, and in particular is oromucosally administered to a human patient. In yet another aspect, the present invention relates to the use of the composition for the oromucosal administration of an active agent according to the invention, or of a dosage form comprising the composition, for the manufacture of a medicament for treatment, and in particular for treating a human patient.

As outlined above, in certain embodiments, the active agent is a GLP-1 receptor agonist. While GLP-1 receptor agonists are approved for the treatment of type 2 diabetes mellitus and, in some cases, obesity, treatment of other indications such as type 1 diabetes mellitus, hypoglycemia, cardiovascular disease, chronic kidney disease, liver diseases, gastrointestinal disorders, metabolic dysfunctions, skeletal muscle and bone conditions, and neurogenerative conditions has been suggested.

Thus, in all of the above embodiments, the method of treatment may comprise a method of treating or preventing diabetes, in particular type 2 diabetes mellitus, a method of treating or preventing obesity, in particular supporting weight management, including weight loss or weight maintenance, or treatment of other (related) conditions such as those outlined above, or selected from the group consisting of hypoglycemia, cardiovascular disease, chronic kidney disease, liver diseases, gastrointestinal disorders, metabolic dysfunctions, skeletal muscle and bone conditions, and neurogenerative conditions.

Also, in a certain embodiment, treatment with the oromucosal therapeutic system according to the invention provides a reduction in at least one active agent-related side effect relative to an equivalent oral (or subcutaneous) dose of the active agent. As outlined above, in certain specific embodiments, such active agent-related side effect is caused by passage through the gastrointestinal system and/or hepatic metabolism. Relative to an equivalent oral dose of the active agent should be understood as a comparison in the incidence and intensity of side effects in a clinical study when using a dose of oromucosal and oral the active agent that leads substantially to the same blood plasma exposure of the active agent. The incidence of the at least one active agent-related side effect relative to an equivalent oral dose of the active agent may be reduced by at least about 30 %, preferably at least about 40 %, more preferably at least about 70 % and most preferably at least about 80 %, and/or the intensity of the at least one active agent-related side effect relative to an equivalent oral dose of the active agent may be reduced. The intensity of a side effect can be determined e.g. by classifying the side effects on a scale indicating "mild", "moderate" or "severe" intensity, and a reduction of the intensity can be quantified by comparing the median intensity.

The composition for the oromucosal administration of an active agent according to the invention, or a dosage form comprising the composition, is preferably administered to a human patient for a period of application of from about 10 minutes to about 5 hours, from about 30 minutes to about 3 hours, from about 45 minutes to about 2 hours, or from about 50 minutes to about 90 minutes.

In any of the treatments outlined for the above aspects and embodiments, the composition for the oromucosal administration of an active agent according to the invention, or a dosage form comprising the composition, is preferably administered by applying the composition or the dosage form to the mucosa of the oral cavity of a human patient and maintained on the mucosa until dissolved. In preferred embodiments, the composition for the oromucosal administration of an active agent according to the invention, or a dosage form comprising the composition, is administered by applying the composition or the dosage form to the buccal, sublingual, gingival or palatal mucosa of the oral cavity of a human patient and maintained on the mucosa until dissolved.

### EXAMPLES

The present invention will now be more fully described with reference to the accompanying examples. It should be understood, however, that the following description is illustrative only and should not be taken in any way as a restriction of the invention. Numerical values provided in the examples regarding the amount of ingredients in the composition or the area weight may vary slightly due to manufacturing variability.

### PRE-EXAMPLES 1A-O

First permeation studies were performed with solutions comprising semaglutide, buserelin, sumatriptan or melatonin, without enhancer, with an enhancer selected from salcaprozate sodium, sodium caprate, sodium glycocholate or an enhancer combination of sodium caprate and sodium glycocholate in different weight ratios of enhancer to active agent.

### Active-containing solution

Artificial saliva is prepared by dissolving 2.0 g potassium cyanate, 14.0 g potassium chloride, 1.8 g sodium dihydrogen phosphate monohydrate, and 0.548 g disodium hydrogen phosphate dihydrate in 1000 ml aqua purificata, adjusting the pH to 7.0 ± 0.05, and diluting the solution 1:10 with aqua purificata. Stock solutions of active in artificial saliva are prepared by:
- dissolving 50.07 mg semaglutide in 50 mL artificial saliva to obtain a semaglutide stock solution with a concentration of 1.0 mg/mL, and
- dissolving sumatriptan in artificial saliva to obtain a sumatriptan stock solution with a concentration of 1.0 mg/mL.

For buserelin, a solution for injection (Profact^{®}, 1 ml solution comprising 1.05 mg buserelin acetate, further excipients: benzyl alcohol, sodium dihydrogenphosphate dihydrate, sodium chloride, sodium hydroxide for adjusting pH, water for injection) with a concentration of 1.0 mg/mL buserelin (free base) was used.

The active-containing solutions to be used for the *in vitro* measurement are prepared by dissolving the enhancer(s) in the solution of active in artificial saliva as indicated in the tables summarizing the corresponding formulation below.

The formulations of the semaglutide-containing solutions of Pre-Examples 1a to 1g based on artificial saliva are summarized in Tables 1.1 and 1.2 below.

**Table 1.1**

| **Ingredient (Trade Name)** | **Pre-Ex. 1a** | **Pre-Ex. 1b** | **Pre-Ex. 1c** | **Pre-Ex. 1d** |
|---|---|---|---|---|
| Semaglutide stock solution (1.0 mg/mL) | N/A (solution used as is) | 2 mL | 4 mL | 4 mL |
| Salcaprozate sodium | - | 120.14 mg | - | - |
| Sodium caprate | - | - | 40.67 mg | 119.59 mg |
| Sodium glycocholate | - | - | - | - |
| | | | | |
| Ratio (active : enhancer 1 : enhancer 2) | - | 1:60:0 | 1:10:0 | 1:30:0 |
| | | | | |

| Parameters of *in vitro* measurement | | | | |
|---|---|---|---|---|
| Volume applied | 250 µl | | | |
| Semaglutide amount applied (regarding 93.6% assay content) | 234.32 µg | | | |
| Diffusion area | 0.6 cm² | | | |
| Semaglutide content | 390.5 µg/cm² | | | |
| applied per area | | | | |

**Table 1.2**

| **Ingredient (Trade Name)** | **Pre-Ex. 1e** | **Pre-Ex. 1f** | **Pre-Ex. 1g** |
|---|---|---|---|
| Semaglutide stock solution (1.0 mg/mL) | 20 mL | 4 mL | 4 mL |
| Sodium caprate | - | - | 119.59 mg |
| Sodium glycocholate | 19.83 mg | 20.59 mg | 21.08 mg |
| | | | |
| Ratio (active : enhancer 1 : enhancer 2) | 1:0:1 | 1:0:5 | 1:30:5 |
| | | | |

| Parameters of *in vitro* measurement | | | |
|---|---|---|---|
| Volume applied | 250 µl | | |
| Semaglutide amount applied (regarding 93.6 % assay content) | 234.32 µg | | |
| Diffusion area | 0.6 cm² | | |
| Semaglutide content applied per area | 390.5 µg/cm² | | |

The formulations of the buserelin-containing solutions of Pre-Examples 1h to 1l are summarized in Table 1.3 below.

**Table 1.3**

| **Ingredient (Trade Name)** | **Pre-Ex. 1h** | **Pre-Ex. 1i** | **Pre-Ex. 1j** | **Pre-Ex. 1k** | **Pre-Ex. 11** |
|---|---|---|---|---|---|
| Buserelin solution for injection, 1.0 mg/mL | Ad 2004.93 mg | Ad 2003.53 mg | Ad 2003.81 mg | N/A (solution used as is) | Ad 1050.82 mg |
| Sodium caprate | 100.53 mg | - | 97.81 mg | - | 51.49 mg |
| Sodium glycocholate | - | 100.01mg | 102.34 mg | - | 52.45 mg |
| | | | | | |
| Ratio (active : enhancer 1: enhancer 2) | 1:50:0 | 1:0:50 | 1:49:51 | 1:0:0 | 1:49:50 |
| | | | | | |

| Parameters of *in vitro* measurement | | | | | |
|---|---|---|---|---|---|
| Volume applied | 500 µl | 500 µl | 500 µl | 200 µl* | 200 µl* |
| Buserelin amount applied | 500 µg | 500 µg | 500 µg | 100 µg | 100 µg |
| Diffusion area | 4.524 cm² | 4.524 cm² | 4.524 cm² | 0.6 cm² | 0.6 cm² |
| Buserelin content applied per area | 111 µg/cm² | 111 µg/cm² | 111 µg/cm² | 167 µg/cm² | 167 µg/cm² |

| | | | | | |
|---|---|---|---|---|---|
| * (100 µl buserelin-containing solution +100 µl artificial saliva) | | | | | |

The formulations of the sumatriptan-containing solutions of Pre-Examples 1m to 1o are summarized in Table 1.4 below.

**Table 1.4**

| **Ingredient (Trade Name)** | **Pre-Ex. 1m** | **Pre-Ex. 1n** | **Pre-Ex. 1o** |
|---|---|---|---|
| Sumatriptan stock solution, 5.00 mg /mL | Ad 2002.81 mg | Ad 2001.75 mg | Ad 2016.91 mg |
| Sodium caprate | 20.1 mg | - | 20.04 mg |
| Sodium glycocholate | - | 20.3 mg | 20.23 mg |
| | | | |
| Ratio (active : enhancer 1: enhancer 2) | 1:10:0 | 1:0:10 | 1:10:10 |
| | | | |

| Parameters of *in vitro* measurement | | | |
|---|---|---|---|
| Volume applied | 500 µl | 500 µl | 500 µl |
| Sumatriptan amount applied | 2500 µg | 2500 µg | 2500 µg |
| Diffusion area | 4.524 cm² | 4.524 cm² | 4.524 cm² |
| Sumatriptan content applied per area | 552 µg/cm² | 552 µg/cm² | 552 µg/cm² |

### Measurement of permeated amount

The permeated amount of the active-containing solutions according to Pre-Examples 1a to 1g and 1k and 1l was determined by *in vitro* experiments in accordance with the OECD Guideline (adopted April 13, 2004) using reconstructed human epithelial tissue inserts (human oral keratinocytes, EpiOral^{™} cell cultures). 6-well Labsolute^{®} cell culture plates were used as acceptor vessels, filled with 1.0 mL of acceptor medium (phosphate buffer solution pH 7.4 with 0.1 % (w/v%) sodium azide as antibacteriological agent). The tissue inserts had a diffusion area of 0.6 cm², and the bottom side of the tissue insert made full contact with the acceptor medium. 250 µl semaglutide-containing solution of each of Pre-Examples 1a to 1g, and 200 µl of a mixture made from 100 µl buserelin-containing solution of each of Pre-Examples 1k and 1l mixed with additional 100 µl of artificial saliva was applied to the top side of the reconstructed tissue. Sampling was performed as full exchange of acceptor medium against fresh and prewarmed medium at sampling points. The permeated amount of the active in the acceptor medium at a temperature of 37 ± 1°C (body temperature) was measured.

The permeated amount of the active-containing solutions according to Pre-Examples 1h to 1j and 1m to 1o was determined by *in vitro* experiments in accordance with the OECD Guideline (adopted April 13, 2004) using porcine mucosal tissue. Mucosal tissue from the inside of the oesophagus from domestic pigs was provided by a local butcher. For preparation of the mucosal tissue, the oesophagus was isolated, cut open lengthwise and separated from the surrounding muscle tissue. The resulting tissue was dermatomised to a nominal thickness of about 400 µm, punched into circular pieces (diameter of 34 mm) and fixated to 6-well cell culture inserts from Greiner Bio One^{®} as diffusion inserts. The resulting diffusion area was 4.524 cm². 6-well Labsolute^{®} cell culture plates were used as acceptor vessels, filled with 1.7mL of acceptor medium (phosphate buffer solution pH 7.4 with 0.1 % (w/v%) sodium azide as antibacteriological agent). The diffusion inserts were placed in the acceptor vessel so that the bottom side of the tissue insert made full contact with the acceptor medium. 500 µl active-containing solution of each of Pre-Examples 1h to 1j and 1m to 1o was applied to the top side of the mounted porcine mucosa tissue in the insert. Sampling was performed as full exchange of acceptor medium against fresh and prewarmed medium at sampling points. The permeated amount of the active in the acceptor medium at a temperature of 37 ± 1°C was measured.

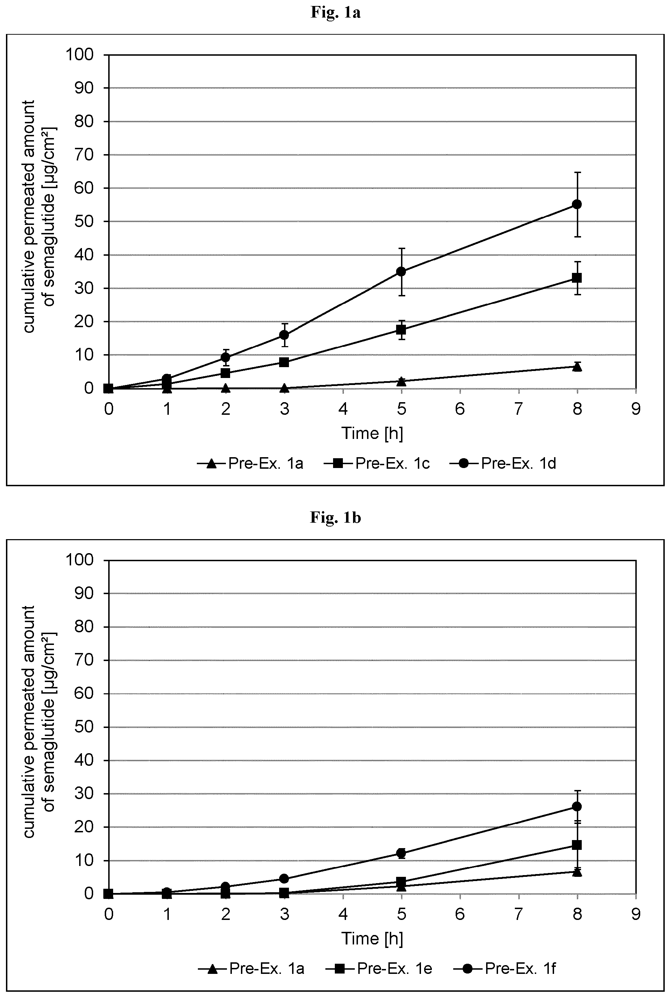

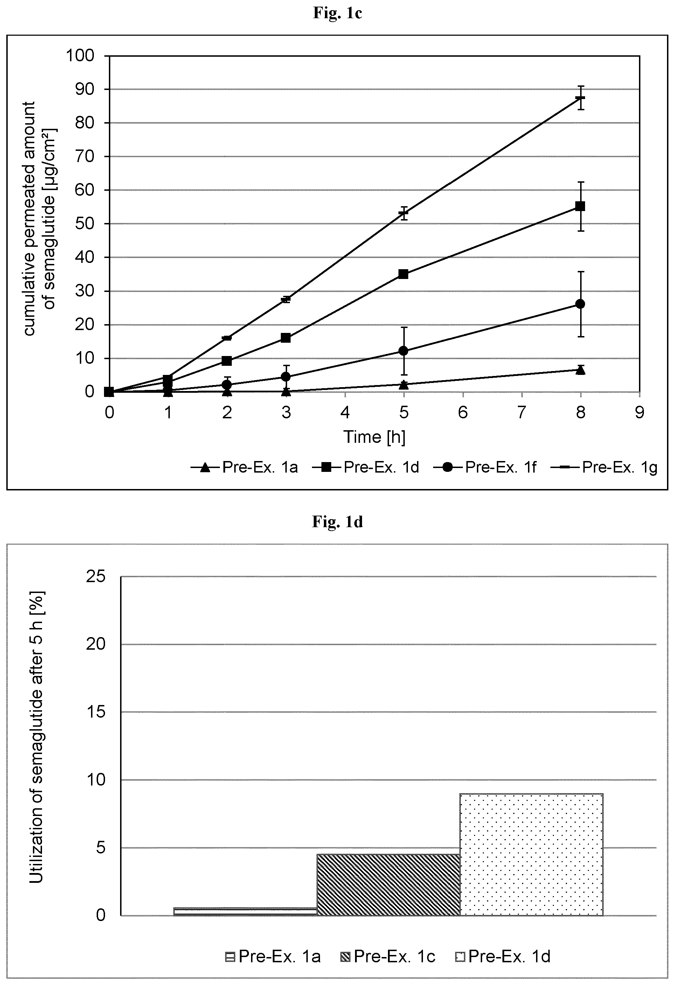

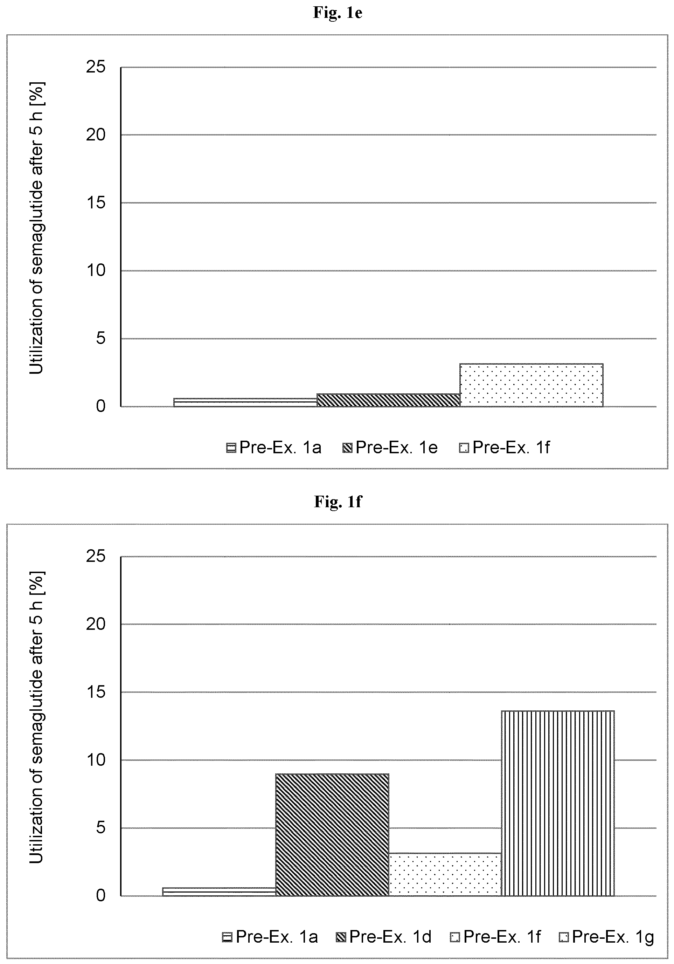

Pre-Example 1b did not show significant permeated amounts of semaglutide. The results of Pre-Examples 1a and 1c to 1g are shown in Tables 1.5 and 1.6 and Figures 1a to 1c. The results of Pre-Examples 1c compared to 1d as well as 1e compared to 1f show that increasing the amount of enhancer from 1:10:0 to 1:30:0 for sodium caprate or from 1:0:1 to 1:0:5 for sodium glycocholate increases the permeated amount of semaglutide, however, not proportionally (less than 100% increase for 3 to 5 times of the enhancer). The results obtained with Pre-Example 1g on the other hand surprisingly showed that the permeated amount could be proportionally increased by combining the increased amounts of enhancer of Pre-Examples 1d and 1f (see Fig. 1c).

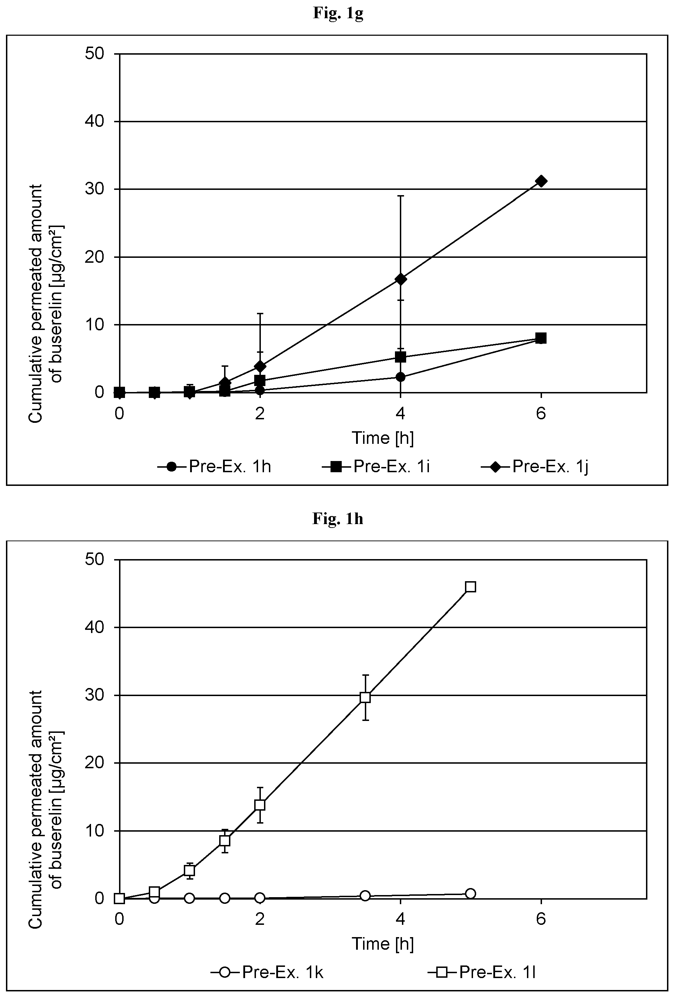

Pre-Example 1j on the other hand showed an increased amount of permeated buserelin obtained with a combination of sodium caprate and sodium glycocholate, which surprisingly surpassed the sum of permeated amounts obtained for each of the enhancers separately with Pre-Examples 1h and 1i (see Fig. 1g). No significant amount of buserelin permeated even with a higher content of active (167 µg/cm²) when measured with human epithelial tissue instead of with porcine mucosa tissue when using a solution without enhancer (Pre-Example 1k), and the permeated amount increased substantially when using the enhancer combination (Pre-Example 1l). The results of Pre-Examples 1h to 1l are shown in Table 1.7 and Figures 1g and 1h.

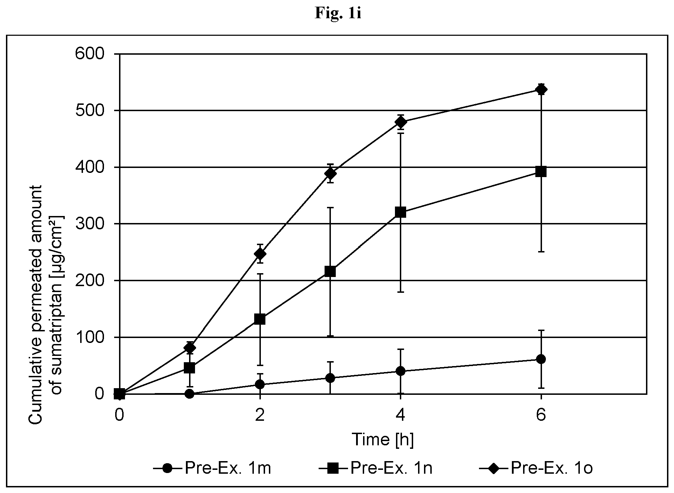

The results of Pre-Examples 1m to 1o are shown in Table 1.8 and Figure 1i and demonstrate that the transmucosal permeation can be also substantially increased for other active agents such as sumatriptan by using the combination of sodium caprate and sodium glycocholate.

**Table 1.5**

| **Cumulative permeated amount of semaglutide with SD* [µg/cm²]** | | | | | | |
|---|---|---|---|---|---|---|
| **Elapsed time [h]** | **Pre-Ex. 1a (n = 3)** | | **Pre-Ex. 1c (n = 3)** | | **Pre-Ex. 1d (n = 3)** | |
| | **Amt** | **SD** | **Amt** | **SD** | **Amt** | **SD** |
| **1** | 0.00 | 0.00 | 0.00 | 0.00 | 0.00 | 0.00 |
| **2** | 0.00 | 0.00 | 1.40 | 0.08 | 2.93 | 1.23 |
| **3** | 0.19 | 0.00 | 4.60 | 0.37 | 9.20 | 2.39 |
| **4** | 0.19 | 0.10 | 7.85 | 1.05 | 16.02 | 3.46 |
| **5** | 2.22 | 0.69 | 17.61 | 2.81 | 35.01 | 7.07 |

| | | | | | | |
|---|---|---|---|---|---|---|
| *: Standard deviation was calculated based on the n-method. | | | | | | |

**Table 1.6**

| **Cumulative permeated amount of semaglutide with SD* [µg/cm²]** | | | | | | |
|---|---|---|---|---|---|---|
| **Elapsed time [h]** | **Pre-Ex. 1e (n = 3)** | | **Pre-Ex. 1f (n = 3)** | | **Pre-Ex. 1g (n = 3)** | |
| | **Amt** | **SD** | **Amt** | **SD** | **Amt** | **SD** |
| **1** | 0.00 | 0.00 | 0.00 | 0.00 | 0.00 | 0.00 |
| **2** | 0.00 | 0.00 | 0.49 | 0.39 | 4.56 | 0.24 |
| **3** | 0.00 | 0.00 | 2.13 | 0.51 | 16.06 | 0.50 |
| **4** | 0.31 | 0.13 | 4.47 | 0.68 | 27.54 | 0.91 |
| **5** | 3.57 | 0.64 | 12.18 | 1.39 | 53.20 | 1.91 |

| | | | | | | |
|---|---|---|---|---|---|---|
| *: Standard deviation was calculated based on the n-method. | | | | | | |

**Table 1.7**

| **Cumulative permeated amount of buserelin with SD* [µg/cm²]** | | | | | | | | | | |
|---|---|---|---|---|---|---|---|---|---|---|
| **Elapsed time [h]** | **Pre-Ex. 1h (n = 3)** | | **Pre-Ex. 1i (n = 3)** | | **Pre-Ex. 1j (n = 3)** | | **Pre-Ex. 1k (n = 3)** | | **Pre-Ex. 1l (n = 3)** | |
| | **Amt** | **SD** | **Amt** | **SD** | **Amt** | **SD** | **Amt** | **SD** | **Amt** | **SD** |
| **0.5** | 0.00 | 0.00 | 0.02 | 0.03 | 0.00 | 0.00 | 0.06 | 0.06 | 0.97 | 0.18 |
| **1** | 0.00 | 0.00 | 0.08 | 0.07 | 0.00 | 0.00 | 0.06 | 0.06 | 4.11 | 0.61 |
| **1.5** | 0.07 | 0.06 | 0.20 | 0.10 | 1.46 | 1.16 | 0.06 | 0.06 | 8.53 | 1.16 |
| **2** | 0.33 | 0.29 | 1.74 | 1.11 | 3.86 | 2.48 | 0.08 | 0.07 | 13.80 | 1.70 |
| **4** | 2.26 | 1.45 | 5.21 | 4.27 | 16.77 | 7.83 | 0.38 | 0.12 | 29.66 | 2.57 |
| **6** | 7.87 | 4.23 | 8.00 | 8.46 | 31.21 | 12.24 | 0.73 | 0.21 | 45.95 | 3.32 |

| | | | | | | | | | | |
|---|---|---|---|---|---|---|---|---|---|---|
| *: Standard deviation was calculated based on the n-method. | | | | | | | | | | |

**Table 1.8**

| **Cumulative permeated amount of sumatriptan with SD* [µg/cm²]** | | | | | | |
|---|---|---|---|---|---|---|
| **Elapsed time [h]** | **Pre-Ex. 1m (n = 2)** | | **Pre-Ex. 1n (n = 3)** | | **Pre-Ex. 1o (n = 3)** | |
| | **Amt** | **SD** | **Amt** | **SD** | **Amt** | **SD** |
| **1** | 0.11 | 0.15 | 46.13 | 33.07 | 81.81 | 10.74 |
| **2** | 16.47 | 19.41 | 131.66 | 80.59 | 247.75 | 16.39 |
| **3** | 27.94 | 28.95 | 216.15 | 112.85 | 389.36 | 15.91 |
| **4** | 40.32 | 39.18 | 320.26 | 140.08 | 479.82 | 12.76 |
| **6** | 61.73 | 50.94 | 392.36 | 140.61 | 538.00 | 8.90 |

| | | | | | | |
|---|---|---|---|---|---|---|
| *: Standard deviation was calculated based on the n-method. | | | | | | |

### Utilization of active

The utilization of semaglutide at 5 hours was calculated based on the cumulative permeated amount at 5 hours and the initial semaglutide content. The results of Pre-Examples 1a and 1c to 1g are shown in Table 1.9 and Figures 1d to 1f.

**Table 1.9**

| **Utilization of semaglutide after 5 hours [%]** | | | | | |
|---|---|---|---|---|---|
| **Pre-Ex. 1a (n = 3)** | **Pre-Ex. 1c (n = 3)** | **Pre-Ex. 1d (n = 3)** | **Pre-Ex. 1e (n = 3)** | **Pre-Ex. 1f (n = 3)** | **Pre-Ex. 1g (n = 3)** |
| **0.57** | **4.51** | **8.97** | **0.91** | **3.12** | **13.62** |

The *in vitro* experiments show that using the combination of two enhancers (sodium caprate and sodium glycocholate) results in increased permeability, adding the single effect of each of the two enhancers, while increasing the amount of one enhancer does not result in linear increase of effect. In addition, the results of Pre-Example 1g are promising with regard to developing semaglutide oromucosal therapeutic systems of useful dimension, as an area of release of approx. 5 cm² would allow to provide the maximum daily dose of 140 µg semaglutide within 3 hours, assuming a similar semaglutide content of about 400 µg/cm².

### PRE-EXAMPLE 2A AND EXAMPLES 2B-D

Further permeation studies were performed with a semaglutide-containing solution comprising an enhancer combination of sodium caprate and sodium glycocholate in a weight ratio of active agent to first permeation enhancer to second permeation enhancer of 1:5:5 and with basic oromucosal therapeutic systems comprising 5.58 wt-% semaglutide and having a weight ratio of active agent to first permeation enhancer to second permeation enhancer of 1:5:5. For the oromucosal therapeutic systems, different contents of semaglutide were tested by using one sample (single dose) or multiple samples stacked on top of each other, namely two samples (double dose) or three samples (triple dose).

### Semaglutide-containing solution (Pre-Example 2a)

Artificial saliva is prepared as described in Example 1 and the semaglutide-containing solution to be used for the *in vitro* measurement is prepared in accordance with the formulation of the solution as specified in Table 2.1 below.

The formulation of the semaglutide-containing solution of Pre-Example 2a based on artificial saliva is summarized in Table 2.1 below.

**Table 2.1**

| **Ingredient (Trade Name)** | **Pre-Ex. 2a** |
|---|---|
| Semaglutide | 30.30 mg |
| Sodium caprate | 150.63 mg |
| Sodium glycocholate | 149.74 mg |
| Artificial saliva | 29926.84 mg |
| | |
| Ratio (active : enhancer 1: enhancer 2) | 1:5:5 |
| | |

| Parameters of *in vitro* measurement | |
|---|---|
| Volume applied | 250 µl |
| Semaglutide amount applied (regarding 93.6% assay content) | 236.34 µg |
| Diffusion area | 0.6 cm² |
| Semaglutide content applied per area | 393.9 µg/cm² |

### Coating composition for oromucosal therapeutic system (Examples 2b, 2c, 2d)

The formulation of the semaglutide-containing coating composition of Examples 2b to 2d is summarized in Table 2.2 below. The formulation is based on weight percent, as also indicated in Table 2.2.

**Table 2.2**

| **Ingredient (Trade Name)** | **Examples 2b, 2c, 2d** | | | |
|---|---|---|---|---|
| | **Amt [g]** | | **Solids [%]** | |
| Semaglutide | 0.40 | | 5.58 | |
| Hydroxy propyl methyl cellulose. Visc.: 3 mPa s (Pharmacoat^{®} 603) | 0.67 | | 9.30 | |
| Hydroxy propyl methyl cellulose. Visc.: 15 mPa s (Pharmacoat^{®} 615) | 1.84 | | 25.57 | |
| Sodium caprate | 2.00 | | 27.78 | |
| Sodium glycocholate | 2.00 | | 27.78 | |
| PEG 400 | 0.29 | | 4.00 | |
| Total | 7.20 | | 100.01 | |
| Area weight [g/m²] | 79.7 | | | |
| Semaglutide content [µg/cm²] | 442.1 | | | |
| Ratio (active : enhancer 1: enhancer 2) | 1:5:5 | | | |
| | | | | |

| | **Ex. 2b** | **Ex. 2c** | | **Ex. 2d** |
|---|---|---|---|---|
| Dose | single | double | | triple |
| (semaglutide content) | | (442.1 µg/cm²) | (884.1 µg/cm²) | (1326.2 µg/cm²) |

### Preparation of the coating composition

For Example 2b, semaglutide and the two enhancers (sodium caprate and sodium glycocholate) were dissolved in aqua purificata while stirring. Hydroxy propyl methyl cellulose (Visc.: 3 mPa s and Visc.: 15 mPa s) and PEG 400 were added consecutively under stirring (420 rpm). The mixture was stirred for approx. 50 min.

### Coating of the coating composition

The resulting semaglutide-containing coating composition according to Example 2b was coated on the non-siliconized side of coating foil paper of 120 g/m² PE2 AB1 (which may function as release liner) and dried in the oven for 16 min at 50°, and in the climate chamber for 60 min at 25° and 60% humidity. Depending on the target area weight the corresponding film applicator gap was kept at approx. 550 µm.

The coating thickness gave an area weight of 79.7 g/m² (Ex. 2b), as determined after normal drying (expecting a residual moisture content of approx. 5 %).

### Preparation of the oromucosal therapeutic system

Individual oromucosal therapeutic systems were punched out from the resulting semaglutide-containing laminate and sealed into pouches of the primary packaging material as conventional in the art.

In specific embodiments, an oromucosal therapeutic system as described above can be provided with a further mucoadhesive layer of larger surface area, preferably with rounded corners, which is free of active agent. This is of advantage when the OTF, on the basis of its physical properties alone, does not adhere sufficiently to the mucosa and/or when the semaglutide-containing layer, for the purpose of avoiding waste, has pronounced corners (square or rectangular shapes).

### Measurement of permeated amount

The permeated amount of the semaglutide-containing solution according to Pre-Example 2a and the oromucosal therapeutic systems prepared according to Examples 2b to 2d were determined by *in vitro* experiments in accordance with the OECD Guideline (adopted April 13, 2004) using reconstructed human epithelial tissue inserts (human oral keratinocytes, EpiOral^{™} cell cultures). 6-well Labsolute^{®} cell culture plates were used as acceptor vessels, filled with 1.0 mL of acceptor medium (phosphate buffer solution pH 7.4 with 0.1 % (w/v%) sodium azide as antibacteriological agent). The tissue inserts had a diffusion area of 0.6 cm², and the bottom side of the tissue insert made full contact with the acceptor medium. For Pre-Example 2a, 250 µl semaglutide-containing solution was applied to the topside of the reconstructed tissue. For Examples 2b to 2d, diecuts with an area of 0.527 cm² were punched from the oromucosal therapeutic system of Example 2b, stacked on top of each other for Example 2c (two diecuts) and Example 2d (three diecuts), as applicable, and applied to the reconstructed tissue with 125 µl artificial saliva each below and above the diecut(s). Sampling was performed as full exchange of acceptor medium against fresh and prewarmed medium at sampling points. The permeated amount of semaglutide in the acceptor medium at a temperature of 37 ± 1°C (body temperature) was measured. The results of Pre-Example 2a and Examples 2b to 2d are shown in Table 2.3 and Figures 2a and 2b.

**Table 2.3**

| **Cumulative permeated amount of semaglutide with SD* [µg/cm²]** | | | | | | | | |
|---|---|---|---|---|---|---|---|---|
| **Elapsed time [h]** | **Pre-Ex. 2a (n = 3)** | | **Ex. 2b (n = 3)** | | **Ex. 2c (n = 3)** | | **Ex. 2d (n = 3)** | |
| | **Amt** | **SD** | **Amt** | **SD** | **Amt** | **SD** | **Amt** | **SD** |
| **1** | 0.35 | 0.06 | 0.21 | 0.15 | 5.64 | 4.23 | 25.45 | 8.05 |
| **2** | 1.59 | 0.58 | 0.95 | 0.57 | 18.05 | 6.95 | 66.00 | 13.29 |
| **3** | 4.19 | 1.32 | 1.91 | 0.99 | 28.53 | 8.69 | 98.09 | 17.64 |
| **4** | 7.30 | 1.95 | 3.33 | 1.40 | 37.59 | 9.69 | 125.57 | 19.91 |
| **5** | 10.75 | 2.73 | 5.13 | 2.01 | 46.24 | 9.93 | 156.36 | 24.30 |

| | | | | | | | | |
|---|---|---|---|---|---|---|---|---|
| *: Standard deviation was calculated based on the n-method. Results are obtained with different lots of oral cell cultures, for which inter-lot variation is generally low enough so that results can be compared. Comparability of the data was also confirmed by internal control samples. | | | | | | | | |

### Utilization of semaglutide

The utilization of semaglutide at 5 hours was calculated based on the cumulative permeated amount at 5 hours and the initial semaglutide content. The results of Pre-Example 2a and Examples 2a to 2d are shown in Table 2.4 and Figure 2c.

**Table 2.4**

| **Utilization of semaglutide after 5 hours [%]** | | | |
|---|---|---|---|
| **Ex. 2a (n = 3)** | **Ex. 2b (n = 3)** | **Ex. 2c (n = 3)** | **Ex. 2d (n = 3)** |
| **2.73** | **1.16** | **5.23** | **11.79** |

The *in vitro* experiments show similar permeability for the artificial saliva solution of Pre-Example 2a and the oromucosal therapeutic system of Example 2b having similar semaglutide contents (about 400 µg/cm²) and a weight ratio of first permeation enhancer to second permeation enhancer to active agent of 5:5:1. Further, Examples 2b to 2d show that, with increasing area weight (and thus increasing active amount), the permeated amount also increases. In particular, the difference in the cumulative permeated amount of semaglutide between single dose, double dose and triple dose is higher than the difference in applied concentration and the correspondingly expected driving force due to the concentration gradient.

### REFERENCE EXAMPLE 3

Further comparative permeation studies were performed with oromucosal therapeutic systems comprising 3.75 wt-% semaglutide and no enhancers.

### Coating composition

The formulation of the semaglutide-containing coating composition of Reference Example 3 is summarized in Table 3.1 below. The formulations are based on weight percent, as also indicated in Table 3.1.

**Table 3.1**

| **Ingredient (Trade Name)** | **Ref. Ex. 3** | |
|---|---|---|
| | **Amt [g]** | **Solids [%]** |
| Semaglutide | 0.29 | 3.77 |
| Hydroxy propyl methyl cellulose. Visc.: 3 mPa s (Pharmacoat^{®} 603) | 2.75 | 36.06 |
| Hydroxy propyl methyl cellulose. Visc.: 80-120 mPa s | 3.16 | 41.40 |
| Hydroxy propyl methyl cellulose. Visc.: 100.000 mPa s (Benecel^{™} K100) | 0.13 | 1.66 |
| Sodium caprate | - | - |
| Sodium glycocholate | - | - |
| PEG400 | 0.29 | 3.83 |
| Glycerine | 0.49 | 6.35 |
| Mint oil | 0.23 | 3.03 |
| Eucalyptol | 0.13 | 1.71 |
| Bitter masker | 0.15 | 1.97 |
| Neotame | 0.02 | 0.22 |
| Total | 7.64 | 100.00 |
| Area weight [g/m²] | 219.5 | |
| Semaglutide content [µg/cm²] | 826.9 | |
| | | |
| Ratio (active : enhancer 1: enhancer 2) | | |

### Preparation of the coating composition

For Reference Example 3, Semaglutide was dissolved in aqua purificata while stirring at approx. 350 rpm. Hydroxy propyl methyl cellulose (Visc.: 3 mPa s, Visc.: 80-120 mPa s and Visc.: 100.000 mPa s), bitter masker and neotame were mixed and added under stirring at approx. 450 rpm. After at least 15 min, PEG 400, glycerine, mint oil and eucalyptol were added consecutively under stirring at approx. 200 rpm. The mixture was stirred for at least 2 hours and left to degas overnight.

### Coating of the coating composition

The resulting semaglutide-containing coating compositions according to Reference Example 3 was coated on the non-siliconized side of coating foil paper of 120 g/m² PE2 AB1 (which may function as release liner) and dried in the oven for 45 min at 50° (normal drying). Depending on the target area weight the corresponding film applicator gap was kept at approx. 1300 µm.

The coating thickness gave an area weight of 219.5 g/m² (Ref. Ex. 3a), as determined after total drying (expecting a residual moisture content of approx. 0%).

### Preparation of the oromucosal therapeutic system

See Example 2.

### Measurement of permeated amount

The permeated amount of the oromucosal therapeutic systems obtained according to Reference Example 3 after normal drying was determined by *in vitro* experiments in accordance with the OECD Guideline (adopted April 13, 2004) using reconstructed human epithelial tissue inserts (human oral keratinocytes, EpiOral^{™} cell cultures). 6-well Labsolute^{®} cell culture plates were used as acceptor vessels, filled with 1.0 mL of acceptor medium (phosphate buffer solution pH 7.4 with 0.1 % (w/v%) sodium azide as antibacteriological agent). The tissue inserts had a diffusion area of 0.6 cm², and the bottom side of the tissue insert made full contact with the acceptor medium. Diecuts with an area of 0.527 cm² were punched from the oromucosal therapeutic systems of Reference Example 3 and applied to the top side of the reconstructed tissue with 125 µl artificial saliva each below and above the diecut. Sampling was performed as full exchange of acceptor medium against fresh and prewarmed medium at sampling points. The permeated amount of semaglutide in the acceptor medium at a temperature of 37 ± 1°C (body temperature) was measured. The results are shown in Table 3.2 and Figure 3a.

**Table 3.2**

| **Cumulative permeated amount of semaglutide with SD* [µg/cm²]** | | |
|---|---|---|
| **Elapsed time [h]** | **Ref. Ex. 3 (n = 3)** | |
| | **Amt** | **SD** |
| **1** | 0.00 | 0.00 |
| **2** | 0.00 | 0.00 |
| **3** | 0.10 | 0.06 |
| **4** | 0.25 | 0.12 |
| **5** | 0.38 | 0.16 |

| | | |
|---|---|---|
| *: Standard deviation was calculated based on the n-method. | | |

### Utilization of semaglutide

The utilization of semaglutide at 5 hours was calculated based on the cumulative permeated amount at 5 hours and the initial semaglutide content. The result is shown in Table 3.3.

**Table 3.3**

| **Utilization of semaglutide after 5 hours [%]** |
|---|
| **Ref. Ex. 3 (n = 3)** |
| **0.00** |

### EXAMPLES 4A-B

Further permeation studies were performed with oromucosal therapeutic systems comprising 3 wt-% semaglutide and having a weight ratio of first permeation enhancer to second permeation enhancer to active agent of 5:5:1, or comprising 3.75 wt-% semaglutide and having a weight ratio of first permeation enhancer to second permeation enhancer to active agent of 4:4:1.

### Coating composition for oromucosal therapeutic system

The formulations of the semaglutide-containing coating compositions of Examples 4a and 4b are summarized in Table 4.1 below. The formulations are based on weight percent, as also indicated in Table 4.1.

**Table 4.1**

| **GLP-1 receptor agonist-containing layer** | | | | |
|---|---|---|---|---|
| **Ingredient (Trade Name)** | **Ex. 4a** | | **Ex. 4b** | |
| | **Amt [g]** | **Solids [%]** | **Amt [g]** | **Solids [%]** |
| Semaglutide | 0.45 | 3.00 | 0.28 | 3.77 |
| Hydroxy propyl methyl cellulose. Visc.: 3 mPa s (Pharmacoat^{®} 603) | 3.91 | 25.98 | 1.71 | 22.83 |
| Hydroxy propyl methyl cellulose. Visc.: 80-120 mPa s | 3.49 | 23.19 | 1.93 | 25.78 |
| Hydroxy propyl methyl cellulose. Visc.: 100.000 mPa s (Benecel^{™} K100) | 0.15 | 1.02 | 0.08 | 1.07 |
| Sodium caprate | 2.25 | 14.97 | 1.13 | 15.02 |
| Sodium glycocholate | 2.26 | 14.99 | 1.13 | 15.06 |
| PEG400 | 0.58 | 3.86 | 0.30 | 4.06 |
| Glycerine | 0.94 | 6.23 | 0.44 | 5.84 |
| Mint oil | 0.43 | 2.86 | 0.22 | 2.90 |
| Eucalyptol | 0.25 | 1.68 | 0.11 | 1.47 |
| Bitter masker | 0.30 | 2.02 | 0.15 | 2.01 |
| Neotame | 0.03 | 0.20 | 0.02 | 0.20 |
| Total | 15.04 | 100.00 | 7.50 | 100.01 |
| Area weight [g/m²] | 209.7 | | 189.9 | |
| Semaglutide content [µg/cm²] | 628.7 | | 716.3 | |
| | | | | |
| Ratio (active : enhancer 1: enhancer 2) | | | 1:5:5 | 1:4:4 |

### Preparation of the coating composition

For Examples 4a and 4b, the two enhancers (sodium caprate and sodium glycocholate) were dissolved in aqua purificata while stirring at approx.200 rpm. Semaglutide was added under stirring at approx. 350 rpm. Hydroxy propyl methyl cellulose (Visc.: 3 mPa s, Visc.: 80 to 120 mPa s and Visc.: 100.000 mPa s), bitter masker and neotame were mixed and added under stirring at approx. 450 rpm. After at least 15 min, PEG 400, glycerine, mint oil and eucalyptol were added consecutively under stirring at approx. 450 rpm. The mixture was stirred for at least 2 hours and left to degas overnight.

### Coating of the coating composition

The resulting semaglutide-containing coating compositions according to Examples 4a and 4b were coated on the non-siliconized side of coating foil paper of 120 g/m² PE2 AB1 (which may function as release liner) and dried in the oven for 45 min at 50° (normal drying). Depending on the target area weight the corresponding film applicator gap was kept at approx. 1300 µm.

The coating thickness gave an area weight of 209.7 g/m² (Ex. 4a) and 189.9 g/m² (Ex. 4b), respectively, as determined after total drying (expecting a residual moisture content of approx. 0%).

### Preparation of the oromucosal therapeutic system

See Example 2.

### Measurement of permeated amount

The permeated amount of the oromucosal therapeutic systems obtained according to Examples 4a and 4b after normal drying was determined by *in vitro* experiments in accordance with the OECD Guideline (adopted April 13, 2004) using reconstructed human epithelial tissue inserts (human oral keratinocytes, EpiOral^{™} cell cultures). 6-well Labsolute^{®} cell culture plates were used as acceptor vessels, filled with 1.0 mL of acceptor medium (phosphate buffer solution pH 7.4 with 0.1 % (w/v%) sodium azide as antibacteriological agent). The tissue inserts had a diffusion area of 0.6 cm², and the bottom side of the tissue insert made full contact with the acceptor medium. Diecuts with an area of 0.527 cm² were punched from the oromucosal therapeutic systems of Examples 4a and 4b and applied to the top side of the reconstructed tissue with 125 µl artificial saliva each below and above the diecut. Sampling was performed at full exchange of acceptor medium against fresh and prewarmed medium at sampling points. The permeated amount of semaglutide in the acceptor medium at a temperature of 37 ± 1°C (body temperature) was measured. The results are shown in Table 4.2 and Figure 4a.

**Table 4.2**

| **Cumulative permeated amount of semaglutide with SD* [µg/cm²]** | | | | |
|---|---|---|---|---|
| **Elapsed time [h]** | **Ex. 4a** | | **Ex. 4b** | |
| | **(n = 3)** | | **(n = 3)** | |
| | **Amt** | **SD** | **Amt** | **SD** |
| **1** | 13.63 | 2.18 | 18.32 | 6.66 |
| **2** | 34.66 | 6.81 | 45.50 | 12.25 |
| **3** | 53.78 | 8.76 | 69.41 | 15.25 |
| **4** | 73.67 | 11.36 | 93.08 | 19.81 |
| **5** | 91.03 | 14.00 | 116.14 | 22.31 |

| | | | | |
|---|---|---|---|---|
| *: Standard deviation was calculated based on the n-method. | | | | |

### Utilization of semaglutide

The utilization of semaglutide at 5 hours was calculated based on the cumulative permeated amount at 5 hours and the initial semaglutide content. The results are shown in Table 4.3 and in Figure 4b.

**Table 4.3**

| **Utilization of semaglutide after 5 hours [%]** | |
|---|---|
| **Ex. 4a (n = 3)** | **Ex. 4b (n = 3)** |
| **14.48** | **16.21** |

### EXAMPLES 5A-5C

Further permeation studies were performed with oromucosal therapeutic systems comprising 3 wt-% or 5 wt-% semaglutide and having a weight ratio of first permeation enhancer to second permeation enhancer to active agent of 4:4:1 or 3:3:1, respectively.

### Coating composition for oromucosal therapeutic system

The formulations of the semaglutide-containing coating compositions of Examples 5a to 5c are summarized in Table 5.1 below. The formulations are based on weight percent, as also indicated in Table 5.1.

**Table 5.1**

| **Ingredient (Trade Name)** | **Ex. 5a** | | **Ex. 5b** | | **Ex. 5c** | |
|---|---|---|---|---|---|---|
| | **Amt [g]** | **Solids [%]** | **Amt [g]** | **Solids [%]** | **Amt [g]** | **Solids [%]** |
| Semaglutide | 0.45 | 3.01 | 0.45 | 3.02 | 0.75 | 4.99 |
| Hydroxy propyl methyl cellulose. Visc.: 3 mPa s (Pharmacoat^{®} 603) | 4.37 | 29.03 | 4.83 | 32.13 | 3.75 | 24.86 |
| Hydroxy propyl methyl cellulose. Visc.: 80-120 mPa s | 3.91 | 25.98 | 4.33 | 28.80 | 3.36 | 22.30 |
| Hydroxy propyl methyl cellulose. Visc.: 100.000 mPa s (Benecel^{™} K100) | 0.18 | 1.18 | 0.19 | 1.29 | 0.15 | 1.00 |
| Sodium caprate | 1.80 | 11.94 | 1.36 | 9.01 | 2.25 | 14.94 |
| Sodium glycocholate | 1.80 | 11.97 | 1.35 | 8.99 | 2.25 | 14.96 |
| PEG400 | 0.61 | 4.04 | 0.59 | 3.94 | 0.60 | 3.97 |
| Glycerine | 0.92 | 6.10 | 0.91 | 6.05 | 0.92 | 6.09 |
| Mint oil | 0.45 | 3.00 | 0.45 | 2.96 | 0.48 | 3.19 |
| Eucalyptol | 0.23 | 1.51 | 0.24 | 1.61 | 0.23 | 1.51 |
| Bitter masker | 0.30 | 2.02 | 0.30 | 2.00 | 0.30 | 1.99 |
| Neotame | 0.03 | 0.22 | 0.03 | 0.20 | 0.03 | 0.21 |
| Total | 15.05 | 100.00 | 15.03 | 100.00 | 15.07 | 100.01 |
| Area weight [g/m²] | 206.5 | | 212.4 | | 203.8 | |
| Semaglutide content [µg/cm²] | 621.4 | | 641.2 | | 1015.9 | |
| | | | | | | |
| Ratio (active : enhancer 1: enhancer 2) | 1:4:4 | | 1:3:3 | | | |

### Preparation of the coating composition

For Examples 5a to 5c, the two enhancers (sodium caprate and sodium glycocholate) were dissolved in aqua purificata while stirring at approx.200 rpm. Semaglutide was added under stirring at approx. 350 rpm. Hydroxy propyl methyl cellulose (Visc.: 3 mPa s, Visc.: 80 to 120 mPa s and Visc.: 100.000 mPa s), bitter masker and neotame were mixed and added under stirring at approx. 450 rpm. After at least 15 min, PEG 400, glycerine, mint oil and eucalyptol were added consecutively under stirring at approx. 450 rpm. The mixture was stirred for at least 2 hours and left to degas overnight.

### Coating of the coating composition

The resulting semaglutide-containing coating compositions according to Examples 5a to 5c were coated on the non-siliconized side of coating foil paper of 120 g/m² PE2 AB1 (which may function as release liner) and dried in the oven for 45 min at 50° (normal drying). Depending on the target area weight the corresponding film applicator gap was kept at approx. 1300 µm.

The coating thickness gave an area weight of 206.5 g/m² (Ex. 5a), 212.4 g/m² (Ex. 5b), and 203.8 g/m² (Ex. 5c), respectively, as determined after total drying (expecting a residual moisture content of approx. 0%).

### Preparation of the oromucosal therapeutic system

See Example 2.

### Measurement of permeated amount

The permeated amount of the oromucosal therapeutic systems obtained according to Examples 5a to 5c after normal drying was determined by *in vitro* experiments in accordance with the OECD Guideline (adopted April 13, 2004) using reconstructed human epithelial tissue inserts (human oral keratinocytes, EpiOral^{™} cell cultures). 6-well Labsolute^{®} cell culture plates were used as acceptor vessels, filled with 1.0 mL of acceptor medium (phosphate buffer solution pH 7.4 with 0.1 % (w/v%) sodium azide as antibacteriological agent). The tissue inserts had a diffusion area of 0.6 cm², and the bottom side of the tissue insert made full contact with the acceptor medium. Diecuts with an area of 0.527 cm² were punched from the oromucosal therapeutic systems of Examples 5a to 5c and applied to the top side of the reconstructed tissue with 125 µl artificial saliva each below and above the diecut. Sampling was performed as full exchange of acceptor medium against fresh and prewarmed medium at sampling points. The permeated amount of semaglutide in the acceptor medium at a temperature of 37 ± 1°C (body temperature) was measured. The results are shown in Table 5.2 and Figure 5a.

**Table 5.2**

| **Cumulative permeated amount of semaglutide with SD* [µg/cm²]** | | | | | | |
|---|---|---|---|---|---|---|
| **Elapsed time [h]** | **Ex. 5a (n = 3)** | | **Ex. 5b (n = 3)** | | **Ex. 5c (n = 3)** | |
| | **Amt** | **SD** | **Amt** | **SD** | **Amt** | **SD** |
| **1** | 11.97 | 2.59 | 0.00 | 0.00 | 26.06 | 14.76 |
| **2** | 24.11 | 5.50 | 3.62 | 1.38 | 63.05 | 27.18 |
| **3** | 56.45 | 13.31 | 14.55 | 1.30 | 73.94 | 29.88 |
| **4** | 86.81 | 19.00 | 24.58 | 2.58 | 85.36 | 33.95 |
| **5** | 112.15 | 21.90 | 33.15 | 3.22 | 96.55 | 36.23 |

| | | | | | | |
|---|---|---|---|---|---|---|
| *: Standard deviation was calculated based on the n-method. | | | | | | |

### Utilization of semaglutide

The utilization of semaglutide at 5 hours was calculated based on the cumulative permeated amount at 5 hours and the initial semaglutide content. The results are shown in Table 5.3 and in Figure 5b.

**Table 5.3**

| **Utilization of semaglutide after 5 hours [%]** | | |
|---|---|---|
| **Ex. 5a (n = 3)** | **Ex. 5b (n = 3)** | **Ex. 5c (n = 3)** |
| **18.05** | **5.17** | **9.50** |

### EXAMPLE 6A, 6B AND REFERENCE EXAMPLE 6C

Further permeation studies were performed with oromucosal therapeutic systems comprising 3 wt-% tirzepatide or liraglutide, respectively, and having a weight ratio of first permeation enhancer to second permeation enhancer to active agent of 5:5:1 (as applicable).

### Coating composition for oromucosal therapeutic system

The formulations of the tirzepatide- or liraglutide-containing coating compositions of Example 6a, 6b and Reference Example 6c are summarized in Table 6.1 below. The formulations are based on weight percent, as also indicated in Table 6.1.

**Table 6.1**

| **Ingredient (Trade Name)** | **Ex. 6a** | | **Ex. 6b** | | **Ref. Ex. 6c** | |
|---|---|---|---|---|---|---|
| | **Amt [g]** | **Solids [%]** | **Amt [g]** | **Solids [%]** | **Amt [g]** | **Solids [%]** |
| Tirzepatide | 0.22 | 3.00 | - | - | - | - |
| Liraglutide | - | - | 0.45 | 2.98 | 0.23 | 3.00 |
| Hydroxy propyl methyl cellulose. Visc.: 3 mPa s (Pharmacoat^{®} 603) | 1.96 | 26.13 | 3.91 | 26.00 | 3.20 | 42.48 |
| Hydroxy propyl methyl cellulose. Visc.: 80-120 mPa s | 1.74 | 23.28 | 3.51 | 23.33 | 2.86 | 38.07 |
| Hydroxy propyl methyl cellulose. Visc.: 100.000 mPa s (Benecel^{™} K100) | 0.08 | 1.01 | 0.15 | 0.98 | 0.12 | 1.57 |
| Sodium caprate | 1.13 | 15.11 | 2.25 | 14.96 | - | - |
| Sodium glycocholate | 1.13 | 15.04 | 2.25 | 14.97 | - | - |
| PEG400 | 0.28 | 3.76 | 0.59 | 3.95 | 0.31 | 4.10 |
| Glycerine | 0.46 | 6.07 | 0.92 | 6.14 | 0.46 | 6.13 |
| Mint oil | 0.22 | 2.89 | 0.44 | 2.92 | 0.22 | 2.92 |
| Eucalyptol | 0.11 | 1.47 | 0.24 | 1.57 | 0.12 | 1.54 |
| Bitter masker | 0.15 | 2.01 | 0.30 | 1.99 | - | - |
| Neotame | 0.02 | 0.21 | 0.03 | 0.21 | 0.01 | 0.20 |
| Total | 7.50 | 99.98 | 14.59 | 100.00 | 7.53 | 100.01 |
| Area weight [g/m²] | 187.1 | | 206.4 | | 230.5 | |
| Tirzepatide content [µg/cm²] | 560.2 | | 613.8 | | 691.0 | |
| | | | | | | |
| Ratio (active : enhancer 1: enhancer 2) | 1:5:5 | | | | - | |

### Preparation of the coating composition

For Examples 6a and 6b, the two enhancers (sodium caprate and sodium glycocholate) were dissolved in aqua purificata while stirring at approx.200 rpm. Tirzepatide or Liraglutide, respectively, was added under stirring at approx. 350 rpm. Hydroxy propyl methyl cellulose (Visc.: 3 mPa s, Visc.: 80-120 mPa s and Visc.: 100.000 mPa s), bitter masker and neotame were mixed and added under stirring at approx. 450 rpm. After at least 15 min, PEG 400, glycerine, mint oil and eucalyptol were added consecutively under stirring at approx. 450 rpm. The mixture was stirred for at least 2 hours and left to degas overnight.

For Reference Example 6c, Liraglutide was dissolved in aqua purificata while stirring at approx. 200 rpm. Hydroxy propyl methyl cellulose (Visc.: 3 mPa s, Visc.: 100 mPa s and Visc.: 100.000 mPa s) and neotame were mixed and added under stirring at approx. 150 rpm. After at least 15 min, PEG 400, glycerine, mint oil and eucalyptol were added consecutively under stirring at approx. 100 rpm. The mixture was stirred for at least 2 hours and left to degas overnight.

### Coating of the coating composition

The resulting tirzepatide-containing coating composition according to Example 6a and the resulting liraglutide-containing coating compositions according to Example 6b and Reference Example 6c were coated on the non-siliconized side of coating foil paper of 120 g/m² PE2 AB1 (which may function as release liner) and dried in the oven for 45 min at 50° (normal drying). Depending on the target area weight the corresponding film applicator gap was kept at approx. 1300 µm.

The coating thickness gave an area weight of 187.1 g/m² (Ex. 6a), 206.4 g/m² (Ex. 6b), and 230.5 g/m² (Ref. Ex. 6c), respectively, as determined after total drying (expecting a residual moisture content of approx. 0%).

### Preparation of the oromucosal therapeutic system

See Example 2.

### Measurement of permeated amount

The permeated amount of the oromucosal therapeutic systems obtained according to Example 6a, 6b and Reference Example 6c after normal drying was determined by *in vitro* experiments in accordance with the OECD Guideline (adopted April 13, 2004) using reconstructed human epithelial tissue inserts (human oral keratinocytes, EpiOral^{™} cell cultures). 6-well Labsolute^{®} cell culture plates were used as acceptor vessels, filled with 1.0 mL of acceptor medium (phosphate buffer solution pH 7.4 with 0.1 % (w/v%) sodium azide as antibacteriological agent). The tissue inserts had a diffusion area of 0.6 cm², and the bottom side of the tissue insert made full contact with the acceptor medium. Diecuts with an area of 0.527 cm² were punched from the oromucosal therapeutic systems of Examples 6a, 6b and Reference Example 6c and applied to the top side of the reconstructed tissue with 125 µl artificial saliva each below and above the diecut. Sampling was performed as full exchange of acceptor medium against fresh and prewarmed medium at sampling points. The permeated amount of semaglutide in the acceptor medium at a temperature of 37 ± 1°C (body temperature) was measured. The results of Example 6a are shown in Table 6.2 and Figure 6a. The results of Example 6b and Reference Example 6c are shown in Table 6.3 and Figure 6b.

**Table 6.2**

| **Cumulative permeated amount of tirzepatide with SD* [µg/cm²]** | | |
|---|---|---|
| **Elapsed time [h]** | **Ex. 6a (n = 3)** | |
| | **Amt** | **SD** |
| **1** | 21.94 | 6.21 |
| **2** | 49.68 | 13.65 |
| **3** | 70.26 | 16.91 |
| **4** | 89.43 | 20.75 |
| **5** | 104.49 | 22.09 |

| | | |
|---|---|---|
| *: Standard deviation was calculated based on the n-method. | | |

**Table 6.3**

| **Cumulative permeated amount of liraglutide with SD* [µg/cm²]** | | | | |
|---|---|---|---|---|
| **Elapsed time [h]** | **Ex. 6b (n = 3)** | | **Ref. Ex. 6c (n = 3)** | |
| | **Amt** | **SD** | **Amt** | **SD** |
| **1** | 4.77 | 3.80 | 0.00 | 0.00 |
| **2** | 12.86 | 9.45 | 0.00 | 0.00 |
| **3** | 20.46 | 13.68 | 0.00 | 0.00 |
| **4** | 27.38 | 16.17 | 0.00 | 0.00 |
| **5** | 31.07 | 17.73 | 0.00 | 0.00 |

| | | | | |
|---|---|---|---|---|
| *: Standard deviation was calculated based on the n-method. Results are obtained with different lots of oral cell cultures, for which inter-lot variation is generally low enough so that results can be compared. Comparability of the data was also confirmed by internal control samples. | | | | |

### Utilization of tirzepatide and liraglutide

The utilization of tirzepatide or liraglutide, respectively, at 5 hours was calculated based on the cumulative permeated amount at 5 hours and the initial tirzepatide or liraglutide content. The results of Example 6a are shown in Table 6.4 and Figure 6c. The results of Example 6b and Reference Example 6c are shown in Table 6.5 and Figure 6d.

**Table 6.4**

| **Utilization of tirzepatide after 5 hours [%]** |
|---|
| **Ex. 6a (n = 3)** |
| **18.62** |

**Table 6.5**

| **Utilization of liraglutide after 5 hours [%]** | |
|---|---|
| **Ex. 6b (n = 3)** | **Ref. Ex. 6c (n = 3)** |
| **5.06** | **0.00** |

### SKIN IRRITATION POTENTIAL EXPERIMENT 7

For prediction of the irritation potential of the enhancers used in artificial saliva solution and oromucosal therapeutic systems, the reduction of the viability of tissues exposed to the enhancers in relation to a negative control was determined using a MTT cell viability assay.

The assay consists of an exposure of reconstructed human epithelial tissue (human oral keratinocytes, EpiOral^{™} cell cultures) to the penetration enhancers, followed by a cell viability test. Cell viability is measured by dehydrogenase conversion of MTT [(3-4,5-dimethyl thiazole 2-yl) 2,5-diphenyltetrazolium-bromide], present in cell mitochondria, into a blue formazan salt that is quantitatively measured after extraction from tissues.

Initial tests with recultivated reconstructed tissue used in the above permeation studies showed that increasing the amount of enhancer generally results in stronger reduction of the cell viability.

In order to predict the irritation potential of the specific enhancer combination (sodium caprate and sodium glycocholate) used in the oromucosal therapeutic systems described above, a MTT effective time (ET-50) test was performed with fresh EpiOral^{™} cell cultures being exposed to an enhancer solution providing the same concentration as a oromucosal therapeutic system comprising 15 wt-% of sodium caprate and of sodium glycocholate.

The solutions used for the MTT ET-50 test are indicate below.

| | |
|---|---|
| Test substance (TS): | 45 mg/ml sodium caprate and 45 mg/ml sodium glycocholate in artificial saliva (corresponding to 15 wt-% each, based on a oromucosal therapeutic system with an area weight of 200 g/m²) |
| Positive control (PC): | 1% Triton X-100 in artificial saliva |
| Negative control (NC): | Artificial saliva |

Relative cell viability is calculated for each tissue as % of the mean of the negative control tissues based on the OD readout value. Skin irritation potential of the test substance is predicted if the remaining relative cell viability is below 50%. The results are shown in Table 7.1 and Figure 7.

**Table 7.1**

| **MTT relative cell viability [%]** | | | |
|---|---|---|---|
| **Elapsed time [min]** | **TS (n = 6)** | **PC (n = 6)** | **NC (n = 6)** |
| **10** | 72.62 | 85.42 | - |
| **20** | 50.67 | - | - |
| **60** | 18.84 | - | 100.00 |
| **120** | 6.86 | 9.84 | - |

The cell viability experiments show that the specific enhancer combination (sodium caprate and sodium glycocholate) has non-critical skin irritation potential (50% viability border was reached after 20 min of exposure). The skin irritation potential can be further reduced by using a lower amount of enhancers.

### The invention relates in particular to the following further embodiments:

1. Composition for the oromucosal administration of an active agent comprising
   A) the active agent, and
   B) a combination of permeation enhancers comprising
      i) a first permeation enhancer selected from the group consisting of C6 to C12 fatty acids and salts thereof; and
      ii) a second permeation enhancer selected from the group consisting of bile acids and salts thereof.
2. Composition for the oromucosal administration of an active agent according to embodiment 1, wherein
   the first permeation enhancer is selected from the group consisting of caproic acid, heptanoic acid, caprylic acid, nonanoic acid, capric acid, undecanoic acid, lauric acid and salts thereof.
3. Composition for the oromucosal administration of an active agent according to embodiment 1 or 2, wherein
   the first permeation enhancer is selected from the group consisting of caproic acid, caprylic acid, capric acid, lauric acid and salts thereof.
4. Composition for the oromucosal administration of an active agent according to embodiment 3, wherein
   the first permeation enhancer is selected from the group consisting of sodium caproate, sodium caprylate, sodium caprate, sodium laurate, potassium caproate, potassium caprylate, potassium caprate, and potassium laurate.
5. Composition for the oromucosal administration of an active agent according to embodiment 4, wherein
   the first permeation enhancer is selected from the group consisting of sodium caprate and potassium caprate.
6. Composition for the oromucosal administration of an active agent according to any one of embodiments 1 to 5, wherein
   the second permeation enhancer is selected from the group consisting of cholic acid, taurocholic acid, glycocholic acid, deoxycholic acid, taurodeoxycholic acid, glycodeoxycholic acid, chenodeoxycholic acid, taurochenodeoxycholic acid, glycochenodeoxycholic acid, and salts thereof.
7. Composition for the oromucosal administration of an active agent according to embodiment 6, wherein
   the second permeation enhancer is selected from the group consisting of sodium cholate, sodium taurocholate, sodium glycocholate, sodium deoxycholate, sodium taurodeoxycholate, sodium glycodeoxycholate, sodium ursodeoxycholate, sodium chenodeoxycholate, sodium taurochenodeoxycholate, sodium glycochenodeoxycholate, sodium cholylsarcosine and sodium N-methyl taurocholate.
8. Composition for the oromucosal administration of an active agent according to embodiment 7, wherein
   the second permeation enhancer is sodium glycocholate.
9. Composition for the oromucosal administration of an active agent according to any one of embodiments 1 to 8, wherein
   if the first permeation enhancer is sodium caprate, the second permeation enhancer is not sodium chenodeoxycholate.
10. Composition for the oromucosal administration of an active agent according to any one of embodiments 1 to 8, wherein
   if the first permeation enhancer is sodium caprate, the second permeation enhancer is selected from the group consisting of sodium cholate, sodium taurocholate, sodium glycocholate, sodium deoxycholate, sodium taurodeoxycholate, sodium glycodeoxycholate, sodium ursodeoxycholate, sodium taurochenodeoxycholate, sodium glycochenodeoxycholate, sodium cholylsarcosine and sodium N-methyl taurocholate.
11. Composition for the oromucosal administration of an active agent according to any one of embodiments 1 to 9, wherein
   if the first permeation enhancer is sodium caprate, the second permeation enhancer is not sodium deoxycholate.
12. Composition for the oromucosal administration of an active agent according to any one of embodiments 1 to 8, wherein
   if the first permeation enhancer is sodium caprate, the second permeation enhancer is selected from the group consisting of sodium cholate, sodium taurocholate, sodium glycocholate, sodium chenodeoxycholate, sodium taurodeoxycholate, sodium glycodeoxycholate, sodium ursodeoxycholate, sodium taurochenodeoxycholate, sodium glycochenodeoxycholate, sodium cholylsarcosine and sodium N-methyl taurocholate.
13. Composition for the oromucosal administration of an active agent according to any one of embodiments 1 to 12, wherein
   the active agent has a solubility in water of more than 0.1 mg/mL, more than 0.5 mg/mL, or more than 1 mg/mL.
14. Composition for the oromucosal administration of an active agent according to any one of embodiments 1 to 13, wherein
   the active agent has a solubility in water of 2 g/mL or less, 1 g/mL or less, or 0.5 g/mL or less.
15. Composition for the oromucosal administration of an active agent according to any one of embodiments 1 to 14, wherein
   the active agent is selected from molecules with a molecular weight of at least 50 g/mol, at least 500 g/mol, at least 1,000 g/mol, or at least 3,000 g/mol, or
   the active agent is selected from molecules with a molecular weight of less than 100,000 g/mol, less than 20,000 g/mol, or less than 5,000 g/mol, or
   the active agent is selected from molecules with a molecular weight of 50 to less than 500 g/mol, of 500 to less than 1,000 g/mol, of 1,000 to less than 3,000 g/mol, of 3,000 to less than 10,000 g/mol, of 10,000 to less than 20,000 g/mol or of 20,000 to less than 100,000 g/mol, or
   the active agent is selected from molecules with a molecular weight of 50 to 100,000 g/mol or less, of 500 to 20,000 g/mol or less, of 1,000 to 10,000 g/mol or less, or of 3,000 to 5,000 g/mol or less.
16. Composition for the oromucosal administration of an active agent according to any one of embodiments 1 to 15, wherein
   the active agent is selected from the group consisting of hormone analogues, opioids, antihistamines, non-steroidal anti-inflammatory agents, anti-emetics, anti-epileptics, vasodilators, anti-tussive agents and expectorants, anti-asthmatics, antacids, anti-spasmodics, antidiabetics, diuretics, anti-hypotensives, antihypertensives, bronchodilators, steroids, antibiotics, antihemorrhoidals, hypnotics, psychotropics, antidiarrheals, mucolytics, sedatives, decongestants, laxatives, vitamins, anti-multiple sclerosis, anti-parkinson, anti-alzheimer, analgesics, stimulants and cannabinoids.
17. Composition for the oromucosal administration of an active agent according to embodiment 16, wherein
   the hormone analogue is an LHRH agonist.
18. Composition for the oromucosal administration of an active agent according to embodiment 17, wherein
   the LHRH agonist is selected from the group consisting of buserelin, nafarelin, leuprolide, goserelin and triptorelin.
19. Composition for the oromucosal administration of an active agent according to embodiment 16, wherein
   the antidiabetic is a GLP-1 receptor agonist.
20. Composition for the oromucosal administration of an active agent according to embodiment 19, wherein
   the GLP-1 receptor agonist is selected from the group consisting of albiglutide, beinaglutide, cotadutide, dulaglutide, efinopegdutide, efocipegtrutide, efpeglenatide, exenatide, liraglutide, lixisenatide, pemvidutide, retatrutide, semaglutide, survodutide, taspoglutide, and tirzepatide.
21. Composition for the oromucosal administration of an active agent according to embodiment 20, wherein
   the GLP-1 receptor agonist is semaglutide.
22. Composition for the oromucosal administration of an active agent according to any one of embodiments 1 to 21, wherein
   the weight ratio of first permeation enhancer to active agent is at least 0.3:1, at least 1:1, at least 2:1 or at least 3:1
23. Composition for the oromucosal administration of an active agent according to any one of embodiments 1 to 22, wherein
   the weight ratio of first permeation enhancer to active agent is 100:1 or less, 70:1 or less, 50:1 or less, or 40:1 or less.
24. Composition for the oromucosal administration of an active agent according to embodiment 22 or 23, wherein
   the weight ratio of first permeation enhancer to active agent is from 0.3:1 to 100:1, from 1:1 to 70:1, from 2:1 to 50:1, or from 3:1 to 40:1.
25. Composition for the oromucosal administration of an active agent according to any one of embodiments 1 to 24, wherein
   the weight ratio of second permeation enhancer to active agent is at least 1.5:1, at least 3:1, at least 10:1, or at least 20:1.
26. Composition for the oromucosal administration of an active agent according to any one of embodiments 1 to 25, wherein
   the weight ratio of second permeation enhancer to active agent is 100:1 or less, 70:1 or less, 50:1 or less, or 40:1 or less.
27. Composition for the oromucosal administration of an active agent according to embodiment 25 or 26, wherein
   the weight ratio of second permeation enhancer to active agent is from 1.5:1 to 100:1, from 3:1 to 70:1, from 10:1 to 50:1, or from 20:1 to 40:1.
28. Composition for the oromucosal administration of an active agent according to any one of embodiments 1 to 27, wherein
   the weight ratio of first permeation enhancer to second permeation enhancer is at least 1:2, or at least 1:1.
29. Composition for the oromucosal administration of an active agent according to any one of embodiments 1 to 28, wherein
   the weight ratio of first permeation enhancer to second permeation enhancer is 10:1 or less, or 6:1 or less.
30. Composition for the oromucosal administration of an active agent according to embodiment 28 or 29, wherein
   the weight ratio of first permeation enhancer to second permeation enhancer is from 1:2 to 10:1, or from 1:1 to 6:1.
31. Composition for the oromucosal administration of an active agent according to any one of embodiments 1 to 30, wherein
   the weight ratio of first permeation enhancer to second permeation enhancer to active agent is about 3:3:1, about 4:4:1, or about 5:5:1 or about 50:50:1.
32. Composition for the oromucosal administration of an active agent according to any one of embodiments 1 to 31, wherein
   the composition is a liquid composition or is a solid composition.
33. Composition for the oromucosal administration of an active agent according to embodiment 32, wherein
   the composition is a solid composition in the form of a powder, granules, or a monolithic structure.
34. Composition for the oromucosal administration of an active agent according to embodiment 33, wherein
   the monolithic structure is a tablet or a film, or is a part of a tablet or a film.
35. Composition for the oromucosal administration of an active agent according to embodiment 34, wherein
   the monolithic structure is a part of a tablet or a film selected from a tablet layer, a tablet core, a tablet shell or a film layer.
36. Composition for the oromucosal administration of an active agent according to any one of embodiments 1 to 35 for use in a method of treatment.
37. Composition for the oromucosal administration of an active agent for use in a method of treating a human patient according to embodiment 36.
38. Use of a composition for the oromucosal administration of an active agent according to any one of embodiments 1 to 35 for the manufacture of a medicament for treatment.
39. Use of a composition for the oromucosal administration of an active agent for the manufacture of a medicament for treating a human patient according to embodiment 38.
40. A method of treatment including oromucosally administering a composition for the oromucosal administration of an active agent according to any one of embodiments 1 to 35.
41. A method of treatment including oromucosally administering a composition for the oromucosal administration of an active agent according to embodiment 40 to a human patient.
42. Oromucosal therapeutic system comprising the composition for the oromucosal administration of an active agent according to any one of embodiments 1 to 35.
43. Oromucosal therapeutic system according to embodiment 42, wherein the oromucosal therapeutic system is in the form of a tablet or a film.

## Claims

1. Composition for the oromucosal administration of an active agent comprising
A) the active agent, and
B) a combination of permeation enhancers comprising
i) a first permeation enhancer selected from the group consisting of C6 to C12 fatty acids and salts thereof; and
ii) a second permeation enhancer selected from the group consisting of bile acids and salts thereof.

2. Composition for the oromucosal administration of an active agent according to claim 1, wherein
the first permeation enhancer is selected from the group consisting of caproic acid, heptanoic acid, caprylic acid, nonanoic acid, capric acid, undecanoic acid, lauric acid and salts thereof, or
the first permeation enhancer is selected from the group consisting of caproic acid, caprylic acid, capric acid, lauric acid and salts thereof, or
the first permeation enhancer is selected from the group consisting of sodium caproate, sodium caprylate, sodium caprate, sodium laurate, potassium caproate, potassium caprylate, potassium caprate, and potassium laurate, or
the first permeation enhancer is selected from the group consisting of sodium caprate and potassium caprate.

3. Composition for the oromucosal administration of an active agent according to any one of claim 1 or 2, wherein
the second permeation enhancer is selected from the group consisting of cholic acid, taurocholic acid, glycocholic acid, deoxycholic acid, taurodeoxycholic acid, glycodeoxycholic acid, chenodeoxycholic acid, taurochenodeoxycholic acid, glycochenodeoxycholic acid, and salts thereof, or
the second permeation enhancer is selected from the group consisting of sodium cholate, sodium taurocholate, sodium glycocholate, sodium deoxycholate, sodium taurodeoxycholate, sodium glycodeoxycholate, sodium ursodeoxycholate, sodium chenodeoxycholate, sodium taurochenodeoxycholate, sodium glycochenodeoxycholate, sodium cholylsarcosine and sodium N-methyl taurocholate, or
the second permeation enhancer is sodium glycocholate.

4. Composition for the oromucosal administration of an active agent according to any one of claims 1 to 3, wherein
if the first permeation enhancer is sodium caprate, the second permeation enhancer is not sodium chenodeoxycholate, or
if the first permeation enhancer is sodium caprate, the second permeation enhancer is selected from the group consisting of sodium cholate, sodium taurocholate, sodium glycocholate, sodium deoxycholate, sodium taurodeoxycholate, sodium glycodeoxycholate, sodium ursodeoxycholate, sodium taurochenodeoxycholate, sodium glycochenodeoxycholate, sodium cholylsarcosine and sodium N-methyl taurocholate.

5. Composition for the oromucosal administration of an active agent according to any one of claims 1 to 4, wherein
if the first permeation enhancer is sodium caprate, the second permeation enhancer is not sodium deoxycholate, or
if the first permeation enhancer is sodium caprate, the second permeation enhancer is selected from the group consisting of sodium cholate, sodium taurocholate, sodium glycocholate, sodium chenodeoxycholate, sodium taurodeoxycholate, sodium glycodeoxycholate, sodium ursodeoxycholate, sodium taurochenodeoxycholate, sodium glycochenodeoxycholate, sodium cholylsarcosine and sodium N-methyl taurocholate.

6. Composition for the oromucosal administration of an active agent according to any one of claims 1 to 5, wherein
the active agent has a solubility in water of more than 0.1 mg/mL, more than 0.5 mg/mL, or more than 1 mg/mL, or
the active agent is selected from molecules with a molecular weight of at least 50 g/mol, at least 500 g/mol, at least 1,000 g/mol, or at least 3,000 g/mol, or
the active agent is selected from molecules with a molecular weight of less than 100,000 g/mol, less than 20,000 g/mol, or less than 5,000 g/mol.

7. Composition for the oromucosal administration of an active agent according to any one of claims 1 to 6, wherein
the active agent is selected from the group consisting of hormone analogues, opioids, antihistamines, non-steroidal anti-inflammatory agents, anti-emetics, anti-epileptics, vasodilators, anti-tussive agents and expectorants, anti-asthmatics, antacids, anti-spasmodics, antidiabetics, diuretics, anti-hypotensives, antihypertensives, bronchodilators, steroids, antibiotics, antihemorrhoidals, hypnotics, psychotropics, antidiarrheals, mucolytics, sedatives, decongestants, laxatives, vitamins, anti-multiple sclerosis, anti-parkinson, anti-alzheimer, analgesics, stimulants and cannabinoids, or
the active agent is an LHRH agonist selected from the group consisting of buserelin, nafarelin, leuprolide, goserelin and triptorelin, or
the active agent is a GLP-1 receptor agonist selected from the group consisting of albiglutide, beinaglutide, cotadutide, dulaglutide, efinopegdutide, efocipegtrutide, efpeglenatide, exenatide, liraglutide, lixisenatide, pemvidutide, retatrutide, semaglutide, survodutide, taspoglutide, and tirzepatide, or
the active agent is semaglutide.

8. Composition for the oromucosal administration of an active agent according to any one of claims 1 to 7, wherein
the weight ratio of first permeation enhancer to active agent is at least 0.3:1, at least 1: 1, at least 2: 1 or at least 3: 1, and/or
the weight ratio of first permeation enhancer to active agent is 100:1 or less, 70:1 or less, 50:1 or less, or 40:1 or less.

9. Composition for the oromucosal administration of an active agent according to any one of claims 1 to 8, wherein
the weight ratio of second permeation enhancer to active agent is at least 1.5:1, at least 3:1, at least 10:1, or at least 20:1, and/or
the weight ratio of second permeation enhancer to active agent is 100:1 or less, 70:1 or less, 50:1 or less, or 40:1 or less.

10. Composition for the oromucosal administration of an active agent according to any one of claims 1 to 9, wherein
the weight ratio of first permeation enhancer to second permeation enhancer is at least 1:2, or at least 1:1, and/or
the weight ratio of first permeation enhancer to second permeation enhancer is 10:1 or less, or 6:1 or less, and/or
the weight ratio of first permeation enhancer to second permeation enhancer to active agent is about 3:3:1, about 4:4:1, or about 5:5:1 or about 50:50:1.

11. Composition for the oromucosal administration of an active agent according to any one of claims 1 to 10, wherein
the composition is a liquid composition or is a solid composition, or
the composition is a solid composition in the form of a monolithic structure, wherein the monolithic structure is a part of a tablet or a film selected from a tablet layer, a tablet core, a tablet shell or a film layer.

12. Composition for the oromucosal administration of an active agent according to any one of claims 1 to 11 for use in a method of treatment.

13. Use of a composition for the oromucosal administration of an active agent according to any one of claims 1 to 11 for the manufacture of a medicament for treatment.

14. A method of treatment including oromucosally administering a composition for the oromucosal administration of an active agent according to any one of claims 1 to 11.

15. Oromucosal therapeutic system comprising the composition for the oromucosal administration of an active agent according to any one of claims 1 to 11.

16. Oromucosal therapeutic system according to claim 15, wherein
the oromucosal therapeutic system is in the form of a tablet or a film.
